(19) **Europäisches Patentamt**
**European Patent Office**
**Office européen des brevets**

(11) **EP 3 867 636 B1**

(12) **EUROPÄISCHE PATENTSCHRIFT**

(45) Veröffentlichungstag und Bekanntmachung des Hinweises auf die Patenterteilung:
**23.11.2022 Patentblatt 2022/47**

(21) Anmeldenummer: **19802076.0**

(22) Anmeldetag: **10.10.2019**

(51) Internationale Patentklassifikation (IPC):
**G01N 29/032** (2006.01)  **G01N 29/22** (2006.01)
**G01N 29/24** (2006.01)

(52) Gemeinsame Patentklassifikation (CPC):
**G01N 29/032; G01F 1/663; G01N 29/024;**
**G01N 29/222; G01N 29/2412; G01P 5/241;**
G01N 2291/017; G01N 2291/02433;
G01N 2291/02836; G01N 2291/0289;
G01N 2291/0425; G01N 2291/044;
G01N 2291/2634

(86) Internationale Anmeldenummer:
**PCT/EP2019/077533**

(87) Internationale Veröffentlichungsnummer:
**WO 2020/078833 (23.04.2020 Gazette 2020/17)**

(54) **VERFAHREN UND VORRICHTUNG ZUR NICHTINVASIVEN BESTIMMUNG VON EIGENSCHAFTEN EINES MULTIPHASENSTROMS**

METHOD AND DEVICE FOR NON-INVASIVE DETERMINATION OF MULTI-PHASE CURRENT PROPERTIES

PROCÉDÉ ET DISPOSITIF DE DÉTERMINATION NON INVASIVE DE CARACTÉRISTIQUES D'UN COURANT POLYPHASÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.10.2018 DE 102018125923**

(43) Veröffentlichungstag der Anmeldung:
**25.08.2021 Patentblatt 2021/34**

(73) Patentinhaber: **Rosen Swiss AG**
**6370 Stans (CH)**

(72) Erfinder:
• **BAUERNSCHMITT, Rüdiger**
**76351 Linkenheim-Hochstetten (DE)**
• **BLACK, Michael**
**48149 Münster (DE)**
• **RODRIGUEZ, Natalia**
**7522 CR Enschede (NL)**
• **SCHLESIGER, Ralf**
**48161 Münster (DE)**
• **REETMEYER, Burkhard**
**49828 Neuenhaus (DE)**

(74) Vertreter: **Wischmeyer, André et al**
**Busse & Busse**
**Patent- und Rechtsanwälte**
**Partnerschaft**
**Großhandelsring 6**
**49084 Osnabrück (DE)**

(56) Entgegenhaltungen:
EP-A2- 1 081 465    EP-A2- 2 913 641
WO-A1-00/03207    WO-A2-2011/078691
WO-A2-2018/175503    JP-A- 2000 097 742
US-A1- 2015 260 561

• AKINORI FURUSAWA ET AL: "Mode control of guided wave in magnetic hollow cylinder using electromagnetic acoustic transducer array", NUCLEAR ENGINEERING AND TECHNOLOGY, Bd. 47, Nr. 2, 1. März 2015 (2015-03-01), Seiten 196-203, XP055686046, ISSN: 1738-5733, DOI: 10.1016/j.net.2014.12.007

EP 3 867 636 B1

**Beschreibung**

Die Erfindung betrifft ein Verfahren und eine Vorrichtung zur nichtinvasiven, Bestimmung von Eigenschaften eines Multiphasenstromes, der einen flüssigen Anteil, insbesondere umfassend Wasser und/oder eine kohlenwasserstoffhaltige Flüssigkeit, und einen gasförmigen Anteil umfasst und durch ein elektrisch leitendes Objekt, vorzugsweise ein Rohr oder eine Pipeline, strömt.

Das Überwachen als Gemisch unterschiedlicher Phasen vorkommender Medien ist beispielsweise im Bereich der Öl- und Gasindustrie relevant, wo u.a. Öl-WasserGas-Gemische gefördert bzw. transportiert werden. Von besonderem Interesse sind dabei die Flussraten der einzelnen Phasen, auf deren Basis beispielsweise Aussagen über die Fördermenge an Öl getroffen werden können. Nichtinvasive Verfahren zeichnen sich dabei dadurch aus, dass die entsprechenden Messvorrichtungen oder Teile davon von außen an die Rohre oder Pipelines angeordnet werden und so den Medientransport nicht beeinträchtigen.

Die zuverlässige Bestimmung von Eigenschaften eines Multiphasenstroms stellt eine technische Herausforderung dar. Insbesondere können zur Bestimmung von Eigenschaften einzelner Anteile oder Phasen des Multiphasenstromes unterschiedliche Messmethoden bzw. Messgeräte besonders geeignet sein. In der Praxis ist die Verwendung mehrerer einzelner Messgeräte jedoch umständlich. Diese müssen beispielsweise hintereinander am Objekt positioniert werden, wodurch viel Bauraum benötigt wird. Auch ist die Installation und Wartung mehrerer Messgeräte zeit- und kostenaufwendig.

Ein weiterer zu beachtender Aspekt bei der Bestimmung von Eigenschaften eines Multiphasenstroms ist, dass unterschiedliche Arten von Strömungen auftreten können, bei denen der gasförmige Anteil und der flüssige Anteil zudem unterschiedliche Flussraten aufweisen können. Bei dem sog. Schichtfluss (stratified flow) fließen die einzelnen Phasen in übereinanderliegenden Schichten. Dies stellt jedoch für die meisten Anwendungen einen eher seltenen Spezialfall dar. Insbesondere bei der Förderung von Rohöl ist sog. Schwallfluss (slug flow) die am häufigsten vorkommende Strömungsart. Dabei wird der gasförmige Anteil überwiegend in großen Blasen - sog. Taylor-Blasen - transportiert, zwischen denen sich den Objektdurchmesser ausfüllende Schwallabschnitte (slugs) des flüssigen Anteils bewegen. Ferner existiert eine Bandbreite von Strömungsarten, die zwischen Schichtfluss und Schwallfluss eingeordnet werden können. Darüber hinaus fließt beim sog. Ringfluss (annular flow) der flüssige Anteil ringförmig an der inneren Objektwand entlang, während der gasförmige Anteil mittig und durch den flüssigen Anteil von der Objektwand beabstandet fließt.

Die WO 2018/175503 A2 offenbart eine Vorrichtung zur Bestimmung der Zusammensetzung, der Fluiddurchflussrate sowie von Dämpfungseffekten bei einem durch eine Rohrleitung strömenden Multiphasenstrom. Dabei werden an je einer Stelle stromauf- und stromabwärts gleichzeitig Ultraschallwellen senkrecht zur Strömungsrichtung eingeschallt und aufgezeichnet. Ausgewählte Eigenschaften des Multiphasenstroms können anschließend beispielsweise durch eine Kreuzkorrelation der Signale ermittelt werden.

Die EP 2 913 641 A2 zeigt eine weitere Vorrichtung zur Durchflussmessung eines Multiphasenstroms als Kombination zweier Durchflussmesser, die einerseits an einem vertikal verlaufenden Rohrleitungsabschnitt und andererseits an einem horizontal verlaufenden Rohrleitungsabschnitt angeordnet werden. Aus den Messsignalen der beiden Durchflussmesser wird die Durchflussrate bestimmt. Ersichtlich kann eine solche Vorrichtung nur dort eingesetzt werden, wo die Rohrleitung sowohl vertikal als auch horizontal verlaufende Abschnitte aufweist.

Aufgabe der vorliegenden Erfindung ist es, ein verbessertes Verfahren und eine verbesserte Vorrichtung für die Bestimmung von Eigenschaften eines Multiphasenstroms bereitzustellen, die insbesondere auch beim Vorliegen von Schwallfluss (slug flow) zuverlässige Ergebnisse liefert. Diese Aufgabe wird durch ein Verfahren nach dem Anspruch 1 gelöst. Ferner wird die Aufgabe durch eine Vorrichtung gemäß Anspruch 24 gelöst. Vorteilhafte Weiterbildungen der Erfindung sind den hierauf rückbezogenen Unteransprüchen zu entnehmen.

Bei einem beispielhaften Verfahren zur nichtinvasiven Bestimmung von Eigenschaften eines Multiphasenstroms, der durch ein elektrisch leitendes Objekt, vorzugsweise ein Rohr oder eine Pipeline, strömt, wird unter Verwendung eines einzigen Setups mit einer Mehrzahl von EMAT-Wandlern zumindest eine Eigenschaft des Multiphasenstroms durch zumindest eine Messmethode bestimmt. Insbesondere wird zumindest eine der folgenden Eigenschaften bestimmt:

- die Geschwindigkeit des gasförmigen Anteils,
- die Geschwindigkeit des flüssigen Anteils,
- der Strömungsquerschnittanteil des gasförmigen Anteils und/oder
- der Wassergehalt im flüssigen Anteil,

wobei statt dem Strömungsquerschnittanteil des gasförmigen Anteils auch der Strömungsquerschnittsanteil des flüssigen Anteils bestimmt werden kann.

Dabei wird zumindest eine der folgenden Messmethoden a) bis e) durchgeführt:

a) Zumindest zwei Signale werden, insbesondere zur Bestimmung der Geschwindigkeit des gasförmigen Anteils, räumlich miteinander korreliert,

b) zumindest ein sich auf Basis einer von einer Reflexionsquelle (22) im Multiphasenstrom reflektierten Welle (28) ergebendes Signal wird, insbesondere zur Bestimmung der Geschwindigkeit des flüssigen Anteils, ausgewertet,

c) zumindest ein sich auf Basis einer stromaufwärts gesendeten, zumindest in einen Teil des Multiphasenstroms eingekoppelten Welle (27) ergebendes Signal und zumindest ein sich auf Basis einer stromabwärts gesendeten, zumindest in einen Teil des Multiphasenstroms eingekoppelten Welle (27) ergebendes Signal werden, insbesondere zur Bestimmung der Geschwindigkeit des flüssigen Anteils, ausgewertet,

d) zumindest ein sich auf Basis einer stromabwärts oder stromaufwärts gesendeten, zumindest in einen Teil des Multiphasenstroms eingekoppelten Welle (27) ergebendes Signal wird, insbesondere zur Bestimmung des Strömungsquerschnittanteils des gasförmigen Anteils und/oder zur Bestimmung des Wassergehalts im flüssigen Anteil (6), ausgewertet,

e) zumindest ein sich auf Basis eines sich ausschließlich in der Objektwand (4) ausbreitenden Teils einer stromabwärts oder stromaufwärts gesendeten Welle (26) ergebendes Signal wird, insbesondere zur Bestimmung des Strömungsquerschnittanteils des gasförmigen Anteils und/oder zur Bestimmung des Wassergehalts im flüssigen Anteil (6), ausgewertet.

Durch eine geschickte Auswahl einer oder vorzugsweise mehrerer Messmethoden wird mit einer relativ geringen Anzahl von EMAT-Wandlern aus einer Gruppe von relevanten Eigenschaften des Multiphasenstroms zumindest eine dieser Eigenschaften zuverlässig bestimmt. Ferner ist die Verwendung eines einzigen EMAT-Setups für alle der möglichen Messmethoden vorgesehen, wodurch das Verfahren besonders praxistauglich ist. Es wird nur wenig Platz und keine Zeit für den Umbau des Setups zur Anwendung weiterer Messmethoden benötigt. Durch die Verwendung von EMAT-Wandlern kann auf eine aufwendige akustische Kopplung mittels insbesondere gelartiger Kopplungsmedien verzichtet werden. Zudem ergeben sich insbesondere in Verbindung mit der Erzeugung von Lamb-Wellen höhere Toleranzen bei der Positionierung der EMAT-Wandler zueinander, was ein Verfahren mit unterschiedlichen Messmethoden, bei dem ein Wandler ggf. mit mehreren Wandlern an verschiedenen Positionen zusammenwirkt, prozesssicherer macht. Insbesondere ist das Verfahren auch bei Vorliegen eines Schwallflusses (slug flow) geeignet, Eigenschaften des Multiphasenflusses zuverlässig zu bestimmen.

Ein an oder nahe der Objektwand angeordneter EMAT-Wandler erzeugt insbesondere als geführte Wellen, vorzugsweise als Lamb-Wellen, ausgebildete Ultraschallwellen in der magnetisierten oder magnetischen Objektwand. Die Wellen breiten sich in der Objektwand axial bzw. parallel zu einer Längsmittelachse des Objekts stromaufwärts und/oder stromabwärts aus. Ein Teil der Ultraschallwellen koppelt unter einem schrägen Winkel als Longitudinalwelle in zumindest einen Teil, beispielsweise den flüssigen oder gasförmigen Anteil oder eine einzige Phase, des Multiphasenstroms ein. Der Winkel, unter dem die Longitudinalwelle einkoppelt, wird durch die Phasengeschwindigkeit der Ultraschallwelle sowie die Schallgeschwindigkeit des Teils des Multiphasenstroms, in den die Longitudinalwelle einkoppelt, bestimmt (Winkelbeziehung). Die Hauptausbreitungsrichtung der Longitudinalwelle weist dabei immer eine Komponente in Richtung stromabwärts oder stromaufwärts auf. Die in den Multiphasenstrom eingekoppelten Longitudinalwellen können, etwa nachdem sie den Strom gequert haben oder von einer Reflexionsquelle im Strom reflektiert wurden, in die Objektwand wiederum als insbesondere als Lamb-Wellen ausgebildete Ultraschallwellen einkoppeln, wobei dies einen der Winkelbeziehung entsprechenden Eintrittswinkel der Longitudinalwelle voraussetzt. Reflexionsquellen sind insbesondere Phasengrenzen im Multiphasenstrom, beispielsweise Gasblasen und/oder Gasbläschen im flüssigen Anteil, Öltropfen im Wasser oder ggf. auch Festkörper. Die in der Objektwand erzeugten oder aus dem Strom eingekoppelten Ultraschallwellen können von demselben oder einem weiteren EMAT-Wandler detektiert werden. Eine solche Ultraschallwelle induziert dabei letztlich über Wirbelströme in der magnetisierten oder magnetischen Objektwand einen Wechselstrom in zumindest einer Leiterbahn des empfangenden EMAT-Wandlers, was ein elektrisches (Empfangs-)Signal ergibt. Im Folgenden wird dort, wo es nicht zwingend notwendig ist, bei Ultraschallwellen nicht spezifiziert, ob es sich um Longitudinalwellen oder etwa um geführte Wellen handelt. Diese werden gemeinsam als "Wellen" bezeichnet. Ferner wird unter einer Welle, die "in den Multiphasenstrom einkoppelt", eine Welle verstanden, die in zumindest einen Teil des Multiphasenstroms einkoppelt. Das Erzeugen einer Welle und die Detektion zumindest eines sich auf Basis dieser Welle ergebenden Signals wird im Folgenden auch als "Puls" bezeichnet. Zur Magnetisierung der Objektwand werden vorzugsweise ein oder mehrere Permanentmagneten verwendet. Alternativ oder ergänzend ist die Verwendung eines oder mehrerer im Vergleich zu den hochfrequenten Wechselfeldern der Wandler quasi-stationären Elektromagneten möglich.

Unter einem "einzigen Setup" ist eine feststehende Anordnung von an oder nahe der Objektwand zu positionierenden bzw. positionierten Wandlern zu verstehen, bei der zur Durchführung unterschiedlicher Messmethoden weder die Anordnung als Ganzes noch einzelne Komponenten der Anordnung örtlich verändert werden müssen. Dies ermöglicht eine einfach handhabbare Bestimmung von Eigenschaften des Multiphasenstroms, wobei ein solches Setup aufgrund der Verwendung der elektromagnetisch-akustischen Wandler (EMAT-Wandler) und deren in Rohrleitungsrichtung in der Regel kurz bauenden Spulen wenig Platz benötigt.

Zur verbesserten Anschaulichkeit werden im Folgenden Ausführungsbeispiele der Erfindung anhand eines Objekts beschrieben, das als horizontal verlaufendes, rundes Rohr ausgebildet ist. Die Erfindung ist jedoch nicht auf derartige Objekte beschränkt.

Insbesondere wird aus zumindest zwei Eigenschaften des Multiphasenstroms zumindest die Flussrate des flüssigen Anteils und/oder die Flussrate des gasförmigen Anteils ermittelt. Somit kann problemlos mit ein und demselben Setup anhand weniger Messmethoden zumindest eine der Flussraten zuverlässig bestimmt werden.

Erfindungsgemäß werden

- zur Bestimmung der Geschwindigkeit des gasförmigen Anteils zumindest zwei Signale räumlich miteinander korreliert,
- zur Bestimmung der Geschwindigkeit des flüssigen Anteils (6) zumindest ein sich auf Basis einer von einer Reflexionsquelle (22) im Multiphasenstrom reflektierten Welle (28) ergebendes Signal ausgewertet und/oder zumindest ein sich auf Basis einer stromaufwärts gesendeten, zumindest in einen Teil des Multiphasenstroms eingekoppelten Welle (27) ergebendes Signal und zumindest ein sich auf Basis einer stromabwärts gesendeten, zumindest in einen Teil des Multiphasenstroms eingekoppelten Welle (27) ergebendes Signal ausgewertet und
- zur Bestimmung des Strömungsquerschnittanteils des gasförmigen Anteils zumindest ein sich auf Basis einer stromabwärts oder stromaufwärts gesendeten, zumindest in einen Teil des Multiphasenstroms eingekoppelten Welle (27) ergebendes Signal ausgewertet und/oder zumindest ein sich auf Basis eines sich ausschließlich in der Objektwand (4) ausbreitenden Teils einer stromabwärts oder stromaufwärts gesendeten Welle (26) ergebendes Signal ausgewertet.

Diese drei Eigenschaften können problemlos mit ein und demselben Setup von EMAT-Wandlern anhand weniger Messmethoden ermittelt werden. Auf Basis dieser Eigenschaften lassen sich insbesondere die Flussrate des gasförmigen Anteils und die Flussrate des flüssigen Anteils bestimmen.

Zur Bestimmung der Geschwindigkeit des flüssigen Anteils wird gemäß einer Ausführung der Erfindung zumindest ein sich auf Basis einer von einer Reflexionsquelle im Multiphasenstrom reflektierten Welle ergebendes Signal ausgewertet werden. Ein an oder nahe der Objektwand auf einer ersten Seite des Objekts angeordneter erster Wandler erzeugt eine sich in der Objektwand axial ausbreitende Welle, wobei ein Teil der Welle unter einem bestimmten Winkel in den Multiphasenstrom einkoppelt. Trifft die in den Multiphasenstrom eingekoppelte Welle auf eine Reflexionsquelle im Multiphasenstrom, wird sie reflektiert. Genügt der Eintrittswinkel der Welle der vorstehend beschriebenen Winkelbeziehung, koppelt die reflektierte Welle in die Objektwand, wobei ein sich auf Basis dieser Welle ergebendes Signal empfangen wird.

Aus dem Signal können Informationen bezüglich der Geschwindigkeit der Reflexionsquelle gewonnen werden. Insbesondere kleine Reflexionsquellen im flüssigen Anteil des Multiphasenstroms, wie beispielsweise Gasbläschen, bewegen sich mit dem flüssigen Anteil mit, so dass aus der Geschwindigkeit derartiger Reflexionsquellen die Geschwindigkeit des flüssigen Anteils ermittelt werden kann.

Vorzugsweise werden periodisch und für einen bestimmten Zeitraum Wellen(-pulse) erzeugt und die sich auf Basis von reflektierten Wellen ergebenden Signale ausgewertet. Wenn die Reflexionsquelle sich nicht bewegt, bleibt eine zeitliche Position bzw. eine Laufzeit des Signals konstant. Aus einer Verschiebung der zeitlichen Position eines auf eine bestimmte Reflexionsquelle zurückgehenden Signals werden Rückschlüsse auf die Geschwindigkeit der Reflexionsquelle gezogen. Hierbei handelt es sich insbesondere um die Geschwindigkeitskomponente senkrecht zur Reflexionsfläche der Reflexionsquelle. Es ist ersichtlich, dass die Wiederholungsfrequenz des Erzeugens von Wellen - im Folgenden auch Pulsfrequenz genannt - ausreichend hoch sein muss. Bei konstanter Pulsfrequenz ist die Verschiebung der zeitlichen Position der Signale im Allgemeinen proportional zur Geschwindigkeit der Reflexionsquelle. Vorzugsweise wird zur Ermittlung, ob zwei Signale von derselben Reflexionsquelle stammen, eine Signalanalyse, insbesondere eine Kreuzkorrelation, durchgeführt. Dabei handelt es sich insbesondere um eine sog. "Pulsed-Doppler-Messung".

Vorzugsweise wird ein sich auf Basis einer von einer senkrecht zur Hauptströmungsrichtung des Multiphasenstroms verlaufenden Reflexionsfläche reflektierten Welle ergebendes Signal ausgewertet. Dieses Signal wird insbesondere von einem weiteren Wandler empfangen, der auf einer der ersten Seite des Objekts gegenüberliegenden Seite an oder nahe der Objektwand angeordnet ist. Eine axiale Geschwindigkeit der Reflexionsquelle, die parallel zur Längsrichtung des Objekts verläuft, wird insbesondere anhand der Beziehung

$$v_\parallel = \frac{1}{2} \Delta t \, f_{PRF} \, c_{ph}$$

ermittelt, wobei $v_\parallel$ die axiale Geschwindigkeit der Reflexionsquelle, $\Delta t$ die zeitliche Verschiebung des Signals, $f_{PRF}$ die

Pulswiederholungsfrequenz und $c_{ph}$ die Phasengeschwindigkeit der Welle im Medium ist.

Alternativ oder zusätzlich wird vorzugsweise ein sich auf Basis einer von einer parallel zur Hauptströmungsrichtung des Multiphasenstroms verlaufenden Reflexionsfläche reflektierten Welle ergebendes Signal ausgewertet. Dieses Signal wird insbesondere von einem auf der ersten Seite des Objekts an oder nahe der Objektwand angeordneten Wandler empfangen. Vorzugsweise ist der erste Wandler ebenfalls als Empfangswandler ausgebildet und empfängt dieses Signal. In einer weiteren erfindungsgemäßen Ausführungsform wird das Signal von einem weiteren Wandler empfangen, der insbesondere in Längsrichtung des Objekts von dem ersten Wandler beabstandet ist. Eine radiale Geschwindigkeit der Reflexionsquelle, die senkrecht zur Längsrichtung des Objekts verläuft, wird insbesondere anhand der Beziehung

$$v_\perp = \frac{1}{2} \Delta t \, f_{PRF} \, c_{ph} \, \frac{1}{\tan\alpha \left(\frac{1}{\sin^2\alpha} - 1\right)}$$

ermittelt, wobei $v_\perp$ die radiale Geschwindigkeit der Reflexionsquelle, $\Delta t$ die zeitliche Verschiebung des Signals, $f_{PRF}$ die Pulswiederholungsfrequenz, $c_{ph}$ die Phasengeschwindigkeit der Welle im Medium und $\alpha$ der Winkel zwischen der Hauptausbreitungsrichtung der in den Multiphasenstrom eingekoppelten Welle und einer senkrecht auf der Objektinnenwand stehenden Lotrechten ist.

Alternativ oder zusätzlich wird vorzugsweise ein sich auf Basis einer von einer senkrecht zur Hauptausbreitungsrichtung der in den Multiphasenstrom eingekoppelten Welle verlaufenden Reflexionsfläche reflektierten Welle ergebendes Signal ausgewertet. Eine derartige Reflexionsfläche bewirkt, dass die in den Multiphasenstrom gekoppelte Welle um 180° zurückgeworfen wird. Üblicherweise verläuft diese Reflexionsfläche weder parallel noch senkrecht zur Längsrichtung des Objekts. In dieser Ausführung hängt die zeitliche Verschiebung des Signals gemäß der Formel

$$v_\parallel + v_\perp \tan\alpha \left(\frac{1}{\sin^2\alpha} - 1\right) = \frac{1}{2} \Delta t \, f_{PRF} \, c_{ph}$$

sowohl von der axialen als auch von der radialen Geschwindigkeitskomponente der Reflexionsquelle ab, wobei $v_\perp$ die radiale Geschwindigkeitskomponente der Reflexionsquelle, $v_\parallel$ die axiale Geschwindigkeitskomponente der Reflexionsquelle, $\Delta t$ die zeitliche Verschiebung des Signals, $f_{PRF}$ die Pulswiederholungsfrequenz, $c_{ph}$ die Phasengeschwindigkeit der Welle im Medium und $\alpha$ der Winkel zwischen der Hauptausbreitungsrichtung der in den Multiphasenstrom eingekoppelten Welle und einer senkrecht auf der Objektinnenwand stehenden Lotrechten ist. Dieses Signal wird insbesondere von einem auf der ersten Seite des Objekts an oder nahe der Objektwand angeordneten Wandler empfangen. Dies kann der erste Wandler sein, sofern dieser zum Empfangen von Signalen ausgebildet ist, oder ein weiterer Wandler sein, der insbesondere in Längsrichtung des Objekts von dem ersten Wandler beabstandet ist.

In Kombination mit der ermittelten Geschwindigkeit einer sich ausschließlich radial bewegenden Reflexionsquelle kann die axiale Geschwindigkeitskomponente isoliert ermittelt werden. Alternativ kann in Kombination mit der ermittelten Geschwindigkeit einer sich ausschließlich axial bewegenden Reflexionsquelle die radiale Geschwindigkeitskomponente isoliert ermittelt werden. Zur Ermittlung sowohl der radialen als auch der axialen Geschwindigkeit von Reflexionsquellen reicht es daher, zwei der drei vorstehend beschriebenen Reflexionsquellenarten zu beobachten.

Vorzugsweise wird zur Ermittlung der Geschwindigkeit der Reflexionsquellen zumindest ein, insbesondere sendender, Wandler an einer untersten Position (6-Uhr-Position) des Objekts angeordnet. Dadurch wird die Wahrscheinlichkeit erhöht, das die Wellen in den flüssigen Anteil einkoppeln, da sich bei den meisten Strömungsarten der überwiegende Teil des gasförmigen Anteils in einem oberen Bereich des Objekts (10-Uhr-Position bis 2-Uhr-Position), insbesondere in einem obersten Bereich des Objekts (12-Uhr-Position), sammelt.

Mit Vorteil werden von dem sendenden Wandler in der Objektwand Lamb-Wellen erzeugt. Diese koppeln als sog. "Lamb-Leckwellen" ("leaky lamb waves") über einen breiten Längsabschnitt der Objektwand in das Medium ein. Dadurch können die sich bewegenden Reflexionsquellen über eine größere Wegstrecke beobachtet werden. Ferner wird mehr Energie für die in das Medium einkoppelnden Longitudinalwellen zur Verfügung gestellt.

Alternativ oder zusätzlich zur Ermittlung der Geschwindigkeit der Reflexionsquelle auf Basis der Verschiebung der zeitlichen Position des Signals wird gemäß einer weiteren Ausführung der Erfindung eine Frequenzverschiebung des sich auf Basis einer von einer Reflexionsquelle im Multiphasenstrom reflektierten Welle ergebenden Signals ausgewertet. Der Doppler-Effekt ermöglicht es aus der Frequenzverschiebung Rückschlusse auf die Geschwindigkeit der Reflexionsquelle zu ziehen. Somit kann die Geschwindigkeit der Reflexionsquelle noch zuverlässiger bestimmt werden.

Dabei handelt es sich insbesondere um eine sog. "Continuous-Wave-Doppler-Messung".

Die vorstehend beschriebene Messmethode zur Bestimmung der Geschwindigkeit des flüssigen Anteils des Multiphasenstroms basierend auf einer Auswertung von sich auf Basis von von einer Reflexionsfläche, die senkrecht oder parallel zur Hauptströmungsrichtung des Multiphasenstroms oder senkrecht zur Hauptausbreitungsrichtung der in den Multiphasenstrom eingekoppelten Welle verläuft, reflektierten Welle ergebenden Signalen kann auch losgelöst von den weiteren beschriebenen Messverfahren als eigenständiges Verfahren durchgeführt werden.

Insbesondere wird diese Methode ebenfalls mit dem nachstehend beschriebenen minimalen Setup von Wandlern durchgeführt. Zur Durchführung verschiedener Messmethoden ist daher keine Neuausrichtung der Wandler nötig, wodurch eine Bestimmung von Eigenschaften des Multiphasenstroms praxistauglicher ist.

Zur Bestimmung der Geschwindigkeit des flüssigen Anteils wird gemäß einer Ausführung der Erfindung zumindest ein sich auf Basis einer stromaufwärts gesendeten, zumindest in einen Teil des Multiphasenstroms eingekoppelten Welle ergebendes Signal und zumindest ein sich auf Basis einer stromabwärts gesendeten, zumindest in einen Teil des Multiphasenstroms eingekoppelten Welle ergebendes Signal ausgewertet werden.

Diese in zumindest einen Teil des Multiphasenstroms eingekoppelten Wellen queren den Multiphasenstrom und koppeln in die Objektwand ein. Aus der Laufzeitdifferenz der beiden Signale wird in einer erfindungsgemäßen Ausführungsform die Geschwindigkeit des flüssigen Anteils bestimmt. Dabei handelt es sich insbesondere um eine sog. "Laufzeitdifferenz-Messung".

Vorzugsweise weisen die Wellen jeweils zumindest einen sich zwischen einer 3-Uhr-Position und einer 9-Uhr-Position erstreckenden Ausbreitungspfad auf. Die Ausbreitungspfade verlaufen vorzugsweise in einer zumindest im Wesentlichen quer zur Richtung der Gravitation verlaufenden Ebene. Dadurch wird die Wahrscheinlichkeit erhöht, das die Wellen den flüssigen Anteil queren, da sich bei den meisten Strömungsarten der überwiegende Teil des gasförmigen Anteils in einem oberen Bereich des Objekts (insbesondere 12-Uhr-Position) sammelt. Im Wesentlichen quer zur Gravitationsrichtung verlaufend ist eine Ebene, wenn ihr Schnittwinkel mit der Gravitationsrichtung 90° ± 10° beträgt.

Eine derartige Konfiguration wird insbesondere durch eine Anordnung von zwei stromaufwärts auf gegenüberliegenden Seiten des Objekts angeordneten Wandlern und zwei stromabwärts auf gegenüberliegenden Seiten des Objekts angeordneten Wandlern realisiert.

Insbesondere wird zur Berechnung der Laufzeiten jeweils ein sich auf Basis eines sich ausschließlich in der Objektwand ausbreitenden Teils der gesendeten Welle ergebenden Signal (direktes Wandsignal) als Referenz verwendet.

Insbesondere wird diese Methode ebenfalls mit dem nachstehend beschriebenen minimalen Setup von Wandlern durchgeführt. Zur Durchführung verschiedener Messmethoden ist daher keine Neuausrichtung der Wandler nötig, wodurch eine Bestimmung von Eigenschaften des Multiphasenstroms praxistauglicher ist.

Zur Bestimmung der Geschwindigkeit des gasförmigen Anteils werden zumindest zwei Signale räumlich miteinander korreliert. An zwei verschiedenen Positionen zeitversetzt empfangene Signale, die sich auf Basis von mit einem Medium wechselwirkenden Wellen ergeben und eine gemeinsame charakteristische Signaleigenschaft, beispielsweise in Form eines spezifischen Signalmusters, aufweisen, können Aufschluss über Geschwindigkeiten im Medium geben. Insbesondere die Geschwindigkeiten einzelner Phasen oder Anteile des Multiphasenstromes können so ermittelt werden.

Vorzugsweise werden zumindest zwei Signale an zwei in Längsrichtung des Objekts voneinander beabstandeten Positionen miteinander korreliert. Insbesondere werden dabei Signale ausgewertet, die auf mit Reflexionsquellen im Multiphasenstrom wechselwirkende Wellen zurückgehen.

Ein an oder nahe der Objektwand auf einer ersten Seite des Objekts angeordneter erster Wandler erzeugt eine sich in der Objektwand axial ausbreitende Welle, wobei ein Teil der Welle unter einem bestimmten Winkel in den Multiphasenstrom einkoppelt. Trifft die in den Multiphasenstrom eingekoppelte Welle auf eine Reflexionsquelle im Multiphasenstrom, wird sie reflektiert. Genügt der Eintrittswinkel der Welle der vorstehend genannten Winkelbeziehung, koppelt sie in die Objektwand ein.

Vorzugsweise wird das sich auf Basis der reflektierten Welle ergebende Signal hinsichtlich zumindest einer Signaleigenschaft, beispielsweise einer Amplitudenschwankung, analysiert. Wird diese Signalcharakteristik an zwei in Längsrichtung des Objekts voneinander beabstandeten Positionen beobachtet, kann anhand der Zeit, die zwischen den beiden Beobachtungen vergangen ist sowie dem Abstand zwischen den beiden Positionen die Geschwindigkeit der zugehörigen Reflexionsquelle bestimmt werden.

Diese Messmethode wird insbesondere durch zwei auf derselben Seite des Objekts angeordnete und in Längsrichtung des Objekts voneinander beabstandete Wandler realisiert. Dabei ist vorzugsweise jeder Wandler zum Senden und Empfangen ausgebildet, so dass die erzeugte Welle und das sich auf Basis der reflektierten Welle ergebende Signal von demselben Wandler gesendet bzw. empfangen wird.

Alternativ wird diese Messmethode gemäß einer weiteren Ausführung der Erfindung durch zwei in Längsrichtung des Objekts voneinander beabstandet angeordnete Wandlerpaare realisiert, die jeweils zumindest einen Sendewandler und einen Empfangswandler aufweisen. Vorzugsweise sind Sendewandler und Empfangswandler dabei bezüglich einer Längsmittelachse des Objekts gegenüberliegend angeordnet.

Insbesondere beträgt der maximale Abstand zwischen den Korrelations-Positionen das Zehnfache des Rohrdurchmessers, bevorzugt das Vierfache des Rohrdurchmessers.

Zur Korrelation zweier Signale werden vorzugsweise verschiedene Signaleigenschaften, wie Frequenz, Amplitude und/oder Phase herangezogen. Insbesondere wird eine Frequenzverschiebung des sich auf Basis einer von einer Reflexionsquelle im Multiphasenstrom reflektierten Welle ergebenden Signals ausgewertet. Vorzugsweise umfasst die Korrelation eine Kreuzkorrelation.

Insbesondere wird diese Methode ebenfalls mit dem nachstehend beschriebenen minimalen Setup von Wandlern durchgeführt. Zur Durchführung verschiedener Messmethoden ist daher keine Neuausrichtung der Wandler nötig, wodurch eine Bestimmung von Eigenschaften des Multiphasenstroms praxistauglicher ist.

Zur Bestimmung des Strömungsquerschnittanteils des gasförmigen Anteils (gas void fraction) wird gemäß einer Ausführung der Erfindung zumindest ein sich auf Basis einer stromabwärts oder stromaufwärts gesendeten, zumindest in einen Teil des Multiphasenstroms eingekoppelten Welle ergebendes Signal ausgewertet.

In Abhängigkeit vom Strömungsquerschnittanteil des gasförmigen Anteils wird die in den Multiphasenstrom eingekoppelten Welle entweder insbesondere an einer parallel zur Hauptströmungsrichtung des Multiphasenstroms verlaufenden Reflexionsfläche reflektiert oder quert den Multiphasenstrom zumindest einmal bzw. wird zumindest einmal durch den Multiphasenstrom transmittiert.

Für den Fall, dass der Multiphasenstrom entlang des Ausbreitungspfades der Welle einen ausreichend großen, zusammenhängenden gasförmigen Anteil aufweist (nicht ausschließliche kleine Gasbläschen) wird sich dieser in der Regel in einem oberen Bereich, insbesondere in der Nähe der 12-Uhr-Position, sammeln. Die Phasengrenze zwischen dem oberen gasförmigen Anteil und dem flüssigen Anteil wird zumindest teilweise eine Menge von Reflexionsquellen mit horizontal verlaufender Reflexionsfläche ausbilden. Vorzugsweise wird der Strömungsquerschnittanteil des gasförmigen Anteils dann über einer Messung der Höhe der Phasengrenze bestimmt. Die Höhe der Phasengrenze wird insbesondere aus der Laufzeit eines sich auf Basis der an einer horizontal verlaufenden Reflexionsfläche reflektierten Welle ergebenden Signals berechnet. Vorzugsweise werden damit größere Gasblasen im Multiphasenstrom, insbesondere Taylor-Blasen bei Vorliegen von Schwallfluss, detektiert. Insbesondere wird zur Berechnung der Laufzeit ein sich auf Basis eines sich ausschließlich in der Objektwand ausbreitenden Teils der gesendeten Welle ergebenden Signal (direktes Wandsignal) als Referenz verwendet.

Für den Fall, dass der Multiphasenstrom entlang zumindest eines Ausbreitungspfades der Welle im Wesentlichen (zumindest bis auf kleine Gasbläschen) aus flüssigem Anteil besteht, kann die Welle den Multiphasenstrom queren und in die Objektwand einkoppeln. Vorzugsweise wird das sich auf Basis der den Multiphasenstrom querenden Welle ergebende Signal ausgewertet. Dieses Signal ist ein Indikator, dass der Strömungsquerschnitt zumindest entlang eines Ausbreitungspfades der Welle vollständig von flüssigem Anteil des Multiphasenstroms eingenommen ist.

Für den Fall, dass der Multiphasenstrom entlang eines größeren Längsabschnitts des Objekts (zumindest bis auf kleine Gasbläschen) aus flüssigem Anteil besteht, kann die von einer ersten Seite der Objektwand in den Multiphasenstrom eingekoppelte Welle diesen zweimal queren, wobei die Welle auf der gegenüberliegenden Seite reflektiert wird und auf der ersten Seite der Objektwand in diese einkoppelt. Vorzugsweise wird das sich auf Basis der den Multiphasenstrom zumindest zweimal querenden Welle ergebende Signal ausgewertet. Dieses Signal ist ein zusätzlicher Indikator, dass der Strömungsquerschnitt zumindest entlang eines Ausbreitungspfades der Welle vollständig von flüssigem Anteil des Multiphasenstroms eingenommen ist, dessen Auswertung eine genauere Bestimmung des Strömungsquerschnittanteils des gasförmigen Anteils ermöglicht.

Insbesondere wird dabei zumindest ein sendender Wandler auf der 6-Uhr-Position angeordnet. Vorzugsweise wird zumindest ein empfangender Wandler auf der 6-Uhr-Position angeordnet. Insbesondere wird zumindest ein empfangender Wandler auf der 12-Uhr-Position angeordnet. Dadurch wird ein zwischen der 6-Uhr-Position und 12-Uhr-Position verlaufender Ausbreitungspfad für die Welle bereitgestellt, so dass der Strömungsquerschnittanteil des gasförmigen Anteils optimal bestimmt werden kann.

Die vorstehend beschriebene Messmethode zur Bestimmung des Strömungsquerschnittanteils des gasförmigen Anteils des Multiphasenstroms basierend auf einer Auswertung von sich auf Basis einer von einer Reflexionsquelle im Multiphasenstrom reflektierten Welle oder einer durch zumindest einen Teil des Multiphasenstroms zumindest einmal transmittierten Welle ergebenden Signalen kann auch losgelöst von den weiteren beschriebenen Messverfahren als eigenständiges Verfahren durchgeführt werden.

Insbesondere wird diese Methode ebenfalls mit dem nachstehend beschriebenen minimalen Setup von Wandlern durchgeführt. Zur Durchführung verschiedener Messmethoden ist daher keine Neuausrichtung der Wandler nötig, wodurch eine Bestimmung von Eigenschaften des Multiphasenstroms praxistauglicher ist.

Zur Bestimmung des Strömungsquerschnittanteils des gasförmigen Anteils (gas void fraction) wird gemäß einer weiteren Ausführung der Erfindung zumindest ein sich auf Basis eines sich ausschließlich in der Objektwand ausbreitenden Teils einer stromabwärts oder stromaufwärts gesendeten Welle ergebendes Signal ausgewertet.

Eine in der Objektwand erzeugte Welle koppelt je nach Zusammensetzung des an die Objektwand angrenzenden Teils des Multiphasenstroms, insbesondere abhängig von der Schallgeschwindigkeit und/oder Dichte in diesem Teil, unterschiedlich gut in den Multiphasenstrom ein. Somit lassen sich insbesondere aus der Abschwächung der Amplitude Rückschlüsse auf die Zusammensetzung des an die Objektwand angrenzenden Teils des Multiphasenstroms ziehen.

Die Zusammensetzung des Multiphasenstroms umfasst insbesondere die Information, ob es sich um einen flüssigen oder gasförmigen Anteil handelt. Ferner umfasst die Zusammensetzung auch Informationen darüber, ob es sich beim flüssigen Anteil um Wasser oder eine kohlenwasserstoffhaltige Flüssigkeit handelt. Der an die Objektwand angrenzende Teil des Multiphasenstroms kann beispielsweise der flüssige Anteil sein, dem als Öl-Wasser-Gemisch mit einem bestimmten Wasseranteil eine Schallgeschwindigkeit des Gemischs zugeordnet ist.

Vorzugsweise wird das zumindest eine sich auf Basis eines sich ausschließlich in der Objektwand ausbreitenden Teils einer an einer ersten Position stromaufwärts oder stromabwärts gesendeten Welle ergebende Signal an einer in Längsrichtung des Objekts von der ersten Position beabstandeten zweiten Position empfangen, wobei anhand der Amplitude des Signals die Zusammensetzung des an die Objektwand angrenzenden Teils des Multiphasenstromes ermittelt wird.

Durch die Ermittlung der Zusammensetzung des Multiphasenstromes an mehreren Umfangspositionen des Objekts werden Rückschlüsse auf den Strömungsquerschnittanteil des gasförmigen bzw. des flüssigen Anteils gewonnen.

Vorzugsweise werden mehrere parallel zur Längsrichtung des Objekts verlaufende Ausbreitungspfade entlang des Objektumfangs bereitgestellt. Insbesondere wird eine - vorzugsweise durch einen das Objekt an einer ersten Position bzw. entlang eines ersten Objektquerschnitts umlaufend umfassenden Wandler erzeugte - Welle stromaufwärts oder stromabwärts vollumfänglich in die Objektwand gesendet, wobei die sich auf Basis des ausschließlich in der Objektwand ausbreitenden Teils dieser Welle ergebenden Signale an einem von dem ersten Objektquerschnitt in Längsrichtung des Objekts beabstandeten zweiten Objektquerschnitt bzw. einer zweiten Position an zumindest zwei verschiedenen Umfangspositionen empfangen werden. Die empfangenen Signale gehen dabei auf eine gemeinsame Welle oder ggf. auf einen gemeinsamen Wellenpuls bzw. Wellenburst zurück und lassen sich daher besser miteinander vergleichen. Alternativ werden gemäß einer weiteren Ausführung der Erfindung mehrere einzeln erzeugte Wellen entlang des Objektumfangs gesendet.

Vorzugsweise werden an der zweiten Position bzw. entlang des zweiten Objektumfangs Signale an zumindest vier, bevorzugt zumindest sechs, besonders bevorzugt zumindest acht, maximal 40, verschiedenen Umfangspositionen empfangen. Daraus lässt sich der Strömungsquerschnittanteil des gasförmigen Anteils zuverlässiger bestimmen.

Zur verbesserten Bestimmung der Zusammensetzung des Multiphasenstroms wird vorzugsweise das Maß der Abschwächung der Amplitude anhand bekannter Zusammensetzungen kalibriert.

Die vorstehend beschriebene Messmethode zur Bestimmung des Strömungsquerschnittanteils des gasförmigen Anteils des Multiphasenstroms basierend auf einer Auswertung von sich auf Basis von sich ausschließlich in der Objektwand ausbreitenden Teilen von stromabwärts oder stromaufwärts gesendeten Wellen ergebenden Signalen kann auch losgelöst von den weiteren beschriebenen Messverfahren als eigenständiges Verfahren durchgeführt werden.

Insbesondere wird diese Methode ebenfalls mit dem nachstehend beschriebenen minimalen Setup von Wandlern durchgeführt. Zur Durchführung verschiedener Messmethoden ist daher keine Neuausrichtung der Wandler nötig, wodurch eine Bestimmung von Eigenschaften des Multiphasenstroms praxistauglicher ist.

Gemäß einer weiteren Ausführung der Erfindung wird zur Bestimmung des Wassergehalts im flüssigen Anteil (sog. water liquid ratio, WLR) zumindest ein sich auf Basis eines sich ausschließlich in der Objektwand ausbreitenden Teils einer stromabwärts oder stromaufwärts gesendeten Welle ergebendes Signal zur Bestimmung des Wassergehalts im flüssigen Anteil ausgewertet.

Eine in der Objektwand erzeugte Welle koppelt je nach Zusammensetzung des an die Objektwand angrenzenden Teils des Multiphasenstroms, insbesondere abhängig von der Schallgeschwindigkeit in diesem Teil, unterschiedlich gut in den Multiphasenstrom ein. Somit lassen sich insbesondere aus der Abschwächung der Amplitude Rückschlüsse auf die Zusammensetzung, insbesondere den Wassergehalt im flüssigen Anteil des Multiphasenstroms, des an die Objektwand angrenzenden Teils des Multiphasenstroms ziehen.

Vorzugsweise werden Sendewandler und Empfangswandler in einem zwischen der 3-Uhr-Position und 9-Uhr-Position, bevorzugt zwischen der 4-Uhr-Position und 8-Uhr-Position, verlaufenden unteren Umfangsbereich angeordnet. Dadurch wird für die meisten Anwendungsfälle gewährleistet, dass die Wandler an jenen Bereichen der Objektwand angeordnet sind, hinter denen sich der flüssige Teil des Multiphasenstroms befindet. Insbesondere wird dabei zumindest ein sendender Wandler auf der 6-Uhr-Position angeordnet. Vorzugsweise wird zumindest ein empfangender Wandler auf der 6-Uhr-Position angeordnet.

Die vorstehend beschriebene Messmethode zur Bestimmung des Wassergehalts im flüssigen Anteil des Multiphasenstroms basierend auf einer Auswertung zumindest eines sich auf Basis von sich ausschließlich in der Objektwand ausbreitenden Teils zumindest einer stromabwärts oder stromaufwärts gesendeten Welle ergebenden Signals kann auch losgelöst von den weiteren beschriebenen Messverfahren als eigenständiges Verfahren durchgeführt werden.

Insbesondere wird diese Methode ebenfalls mit dem nachstehend beschriebenen minimalen Setup von Wandlern durchgeführt. Zur Durchführung verschiedener Messmethoden ist daher keine Neuausrichtung der Wandler nötig, wodurch eine Bestimmung von Eigenschaften des Multiphasenstroms praxistauglicher ist.

Gemäß einer weiteren Ausführung der Erfindung wird zur Bestimmung des Wassergehalts im flüssigen Anteil zumindest ein sich auf Basis einer stromaufwärts oder stromabwärts gesendeten, zumindest in einen Teil des Multiphasenstroms eingekoppelten Welle ergebendes Signal ausgewertet. Vorzugsweise wird ein sich auf Basis der den Multiphasenstrom, insbesondere dessen flüssigen Anteil, querenden Welle ergebendes Signal ausgewertet.

In einer erfindungsgemäßen Ausführungsform wird aus der Laufzeit eines sich auf Basis einer den flüssigen Anteil querenden Welle ergebenden Signals die Schallgeschwindigkeit des flüssigen Anteils ermittelt. Unter Kenntnis der Schallgeschwindigkeiten der einzelnen Komponenten des flüssigen Anteils, d.h. des Wassers und/oder der kohlenwasserstoffhaltigen Flüssigkeit, kann der jeweilige Anteil der Komponente und somit der Wassergehalt im flüssigen Anteil ermittelt werden.

Insbesondere wird zur Berechnung der Laufzeit ein sich auf Basis eines sich ausschließlich in der Objektwand ausbreitenden Teils der gesendeten Welle ergebenden Signal (direktes Wandsignal) als Referenz verwendet.

Vorzugsweise weisen die Wellen jeweils zumindest einen sich zwischen einer 3-Uhr-Position und einer 9-Uhr-Position erstreckenden Ausbreitungspfad auf. Die Ausbreitungspfade verlaufen insbesondere in einer im Wesentlichen quer zur Richtung der Gravitation verlaufenden Ebene. Dadurch wird die Wahrscheinlichkeit erhöht, das die Wellen den flüssigen Anteil queren, da sich bei den meisten Strömungsarten der überwiegende Teil des gasförmigen Anteils in einem oberen Bereich des Objekts (10-Uhr-Position bis 2-Uhr Position), insbesondere in ein einer obersten Position (12-Uhr-Position), sammelt.

Eine derartige Konfiguration wird insbesondere durch eine Anordnung von zwei in Längsrichtung des Objekts beabstandeten Wandlern auf zwei gegenüberliegenden Seiten des Objekts realisiert. Vorzugsweise ist auf derselben Seite, auf der der Sendewandler angeordnet ist, ein weiterer Empfangswandler angeordnet, um ein direktes Wandsignal zu empfangen.

Vorzugsweise wird der Wassergehalt im flüssigen Anteil zum einen mittels einer stromabwärts gesendeten Welle und zum anderen mittels einer stromaufwärts gesendeten Welle bestimmt. Demnach wird zur Bestimmung des Wassergehalts im flüssigen Anteil zumindest ein sich auf Basis einer stromaufwärts oder stromabwärts gesendeten, zumindest in einen Teil des Multiphasenstroms eingekoppelten Welle ergebendes Signal und ein sich auf Basis einer weiteren in die jeweils andere Richtung (stromaufwärts oder stromabwärts) gesendeten, zumindest in einen Teil des Multiphasenstroms eingekoppelten Welle ergebendes Signal ausgewertet. Aus der Kombination der beiden Ergebnisse kann der Wassergehalt im flüssigen Anteil genauer bestimmt werden. Eine derartige Konfiguration wird insbesondere durch eine Anordnung von zwei stromaufwärts auf gegenüberliegenden Seiten des Objekts angeordneten Wandlern und zwei stromabwärts auf gegenüberliegenden Seiten des Objekts angeordneten Wandlern realisiert. Insbesondere wird diese Methode ebenfalls mit dem nachstehend beschriebenen minimalen Setup von Wandlern durchgeführt. Zur Durchführung verschiedener Messmethoden ist daher keine Neuausrichtung der Wandler nötig, wodurch eine Bestimmung von Eigenschaften des Multiphasenstroms praxistauglicher ist.

In einer bevorzugten Ausführungsform der Erfindung sind zumindest einer der Messmethoden Konfigurationsparameter zugeordnet, anhand dessen die für die Messmethode notwendigen Wandler gesteuert werden. Anhand der Konfigurationsparameter wird insbesondere bestimmt, welche Wandler zu welchem Zeitpunkt, mit welcher Pulswiederholungsfrequenz, puls- oder burstartig, mit welcher Frequenz und/oder mit welcher Amplitude senden. Dadurch können effizient unterschiedliche Messmethoden parallel oder sequentiell durchgeführt werden. Ferner wird anhand der Konfigurationsparameter insbesondere bestimmt welche Wandler in welchen Zeitintervallen empfangen, wobei vorzugsweise das Empfangen erst nach Ablauf einer vorgegebenen Verzögerungszeit nach Senden des zu empfangenen Signals beginnt. Dadurch kann insbesondere bei der parallelen Durchführung unterschiedlicher Messmethoden ein gezielteres Empfangen von Signalen gewährleistet werden.

Gemäß einer weiteren bevorzugten Ausgestaltung der Erfindung wird zumindest einer der Wandler sowohl als Sender als auch als Empfänger eingesetzt. Dadurch kann die Anzahl der zur Durchführung des Verfahrens notwendigen Wandler reduziert werden, wodurch das Verfahren praxistauglicher ist. Vorzugsweise sind alle Wandler derart ausgebildet, dass die sowohl als Sender als auch als Empfänger einsetzbar sind.

In einer weiteren bevorzugten Ausführungsform der Erfindung sendet einer der Wandler gerichtet stromaufwärts und/oder stromabwärts. Die Aussendung von Wellen erfolgt somit zielgerichtet nach der jeweiligen Notwendigkeit der Messmethode.

So können Wellen beispielsweise überwiegend in Richtung eines Empfängers gesendet werden. Entlang anderer Ausbreitungspfade werden hingegen keine oder kaum Wellen gesendet, wodurch auch ein besseres Signal-Rausch-Verhältnis erreicht wird. Dazu ist der sendende EMAT-Wandler vorzugsweise als Phased-Array-Wandler ausgebildet. Bevorzugt sind alle Wandler als Phased-Array-Wandler ausgebildet.

Gemäß einer weiteren bevorzugten Ausgestaltung der Erfindung erzeugt zumindest einer der Wandler Lamb-Wellen in der Objektwand. Lamb-Wellen koppeln als sog. "Lamb-Leckwellen" ("leaky lamb waves") über einen breiten Längsabschnitt der Objektwand in das Medium ein und können daher über den gesamten Längsabschnitt mit diesem wechselwirken. Insbesondere sich bewegende Reflexionsquellen im Multiphasenstrom sind auf diese Weise besser zu detektieren.

In einer weiteren bevorzugten Ausführungsform der Erfindung wird für zumindest eine Eigenschaft des Multiphasenstromes die diese bestimmende Messmethode in Abhängigkeit des Verhältnisses von gasförmigem Anteil zu flüssigem Anteil des Multiphasenstromes ausgewählt. Vorzugsweise wird für zumindest eine Eigenschaft des Multiphasenstromes die diese bestimmende Messmethode in Abhängigkeit der Flussrate des flüssigen Anteils und/oder der Flussrate des gasförmigen Anteils ausgewählt.

Mit steigendem Gehalt an gasförmigem Anteil können Wellen den Multiphasenstrom schlechter queren, so dass darauf basierende Messmethoden nur begrenzt funktionieren. Vorzugsweise wird daher zur Bestimmung der Geschwindigkeit des flüssigen Anteils und/oder zur Bestimmung des Wassergehalts im flüssigen Anteil bei steigendem Gehalt an gasförmigem Anteil auf Messmethoden, die auf einer Transmission der Wellen durch den Multiphasenstrom basieren, verzichtet und stattdessen eine weitere Messmethode eingesetzt. Insbesondere wird alternativ die weitere Messmethode zur Bestimmung der jeweiligen Eigenschaft zusätzlich durchgeführt und die Ergebnisse der auf einer Transmission der Wellen basierenden Messmethode und der weiteren Messmethode zusammen ausgewertet. Dies gilt insbesondere bei einem GVF-Wert größer gleich 50%.

Mit sinkendem Gehalt an gasförmigem Anteil sinkt auch die Anzahl der Reflexionsquellen im Multiphasenstrom, so dass Messmethoden, bei denen sich auf Basis von an Reflexionsquellen reflektierten Wellen ergebene Signale ausgewertet werden, nur begrenzt funktionieren. Vorzugsweise wird daher zur Bestimmung der Geschwindigkeit des flüssigen Anteils und/oder zur Bestimmung des Strömungsquerschnittanteils des gasförmigen Anteils bei sinkendem Gehalt an gasförmigem Anteil auf Messmethoden, bei denen sich auf Basis von an Reflexionsquellen reflektierten Wellen ergebene Signale ausgewertet werden, verzichtet und stattdessen eine weitere Messmethode eingesetzt. Insbesondere wird alternativ die weitere Messmethode zur Bestimmung der jeweiligen Eigenschaft zusätzlich durchgeführt und die Ergebnisse der auf einer Transmission der Wellen basierenden Messmethode und der weiteren Messmethode zusammen ausgewertet. Dies gilt insbesondere bei einem GVF-Wert kleiner 50%.

Durch die Verwendung zweier unterschiedlicher, sich insbesondere ergänzender Methoden ist eine zuverlässige Bestimmung der jeweiligen Eigenschaft bei unterschiedlichen Gegebenheiten gewährleistet. Insbesondere ist es durch die Verwendung des nachstehend beschriebenen Setups problemlos möglich, zwischen unterschiedlichen, sich insbesondere ergänzenden, Messmethoden zu wählen, ohne einzelne Wandler oder das gesamte Setup neu auszurichten.

Gemäß einer weiteren bevorzugten Ausgestaltung der Erfindung wird zur Bestimmung zumindest einer der Eigenschaften für zumindest eine Messmethode eine Mehrzahl von Einzelmessungen durchgeführt und ausgewertet, wobei insbesondere ein Mittelwert und/oder ein Maximum ermittelt wird. So können insbesondere schwankende Messwerte über einen längeren Zeitraum aufgezeichnet werden und Eigenschaften des Multiphasenstroms zuverlässig bestimmt werden. Insbesondere bei Vorliegen von Schwallfluss unterliegen Eigenschaften wie beispielsweise die Geschwindigkeit des flüssigen Anteils oder der Strömungsquerschnitt des gasförmigen Anteils relativ starken Schwankungen, so dass diese vorzugsweise zumindest über eine Mehrzahl von Schwalldurchgängen anhand einer Mehrzahl von Einzelmessungen bestimmt werden und die Ergebnisse anschließend zur Bildung eines (zeitlichen) Mittelwerts herangezogen werden.

In einer weiteren bevorzugten Ausführungsform der Erfindung wird für zumindest eine Messmethode eine Pulswiederholungsfrequenz von zumindest 200 Hz, bevorzugt zumindest 400 Hz, besonders bevorzugt zumindest 800 Hz, und maximal 5 kHz verwendet. Somit werden insbesondere stark schwankende Eigenschaften des Multiphasenstroms ausreichend zeitaufgelöst bestimmt, so dass ein so ein zuverlässiger Mittelwert gebildet werden kann. Ferner können durch unterschiedliche parallel oder sequentiell durchgeführte Messmethoden mehrere Eigenschaften bestimmt werden, die aufgrund der zeitlichen Nähe ihrer Ermittlung miteinander verrechnet oder in anderer Weise in Beziehung gesetzt werden können. Die jeweilige Pulswiederholungsfrequenz für die jeweilige Messmethode hängt insbesondere von der Schwankung einzelner oder mehrerer Eigenschaften ab. Bevorzugt wird eine derartige Pulswiederholungsfrequenz dann verwendet, wenn zur Bestimmung der Geschwindigkeit des flüssigen Anteils zumindest ein sich auf Basis einer von einer Reflexionsquelle im Multiphasenstrom reflektierten Welle ergebendes Signal ausgewertet wird. Insbesondere wird bei anderen Messmethoden eine Pulswiederholungsfrequenz von 1 Hz bis 50 Hz, bevorzugt von 5 Hz bis 25 Hz, verwendet.

Insbesondere wird bei der Auswertung von zeitlichen Positionen von Signalen, beispielsweise zur Ermittlung von Laufzeitdifferenzen, ein sich auf Basis eines sich ausschließlich in der Objektwand ausbreitenden Teils einer stromabwärts oder stromaufwärts gesendeten Welle ergebendes Signal als Bezugs- bzw. Referenzsignal verwendet.

Die Flussrate $Q_L$ des flüssigen Anteils wird insbesondere anhand folgender Formel ermittelt

$$Q_L = A\overline{v_L}(1 - \overline{\varepsilon_G}),$$

wobei A die Querschnittsfläche des Objekts, $\overline{v_L}$ die gemittelte Geschwindigkeit des flüssigen Anteils und $\overline{\varepsilon_G}$ der gemittelte Strömungsquerschnittanteil des gasförmigen Anteils ist.

Die Flussrate $Q_G$ des gasförmigen Anteils wird insbesondere anhand folgender Formel ermittelt

$$Q_G = A\overline{v_G}\,\overline{\varepsilon_G},$$

wobei A die Querschnittsfläche des Objekts, $\overline{v_G}$ die gemittelte Geschwindigkeit des gasförmigen Anteils und $\varepsilon_G$ der gemittelte Strömungsquerschnittanteil des gasförmigen Anteils ist.

Die Gesamtflussrate $Q_T$ wird somit insbesondere durch folgende Relation ermittelt:

$$Q_T = Q_F + Q_G$$

Der Anteil des Flusses des gasförmigen Anteils an der Gesamtflussrate (sog. gas volume fraction, GVF) wird insbesondere folgendermaßen ermittelt:

$$GVF = \frac{Q_G}{Q_F + Q_G} = \frac{Q_G}{Q_T}$$

Gemäß dem bereits vor- sowie dem weiter unten nachbeschrieben wird die eingangs gestellte Aufgabe auch durch eine Vorrichtung zur nichtinvasiven Bestimmung von Eigenschaften eines Multiphasenstromes, der einen flüssigen Anteil, insbesondere umfassend Wasser und/oder eine kohlenwasserstoffhaltige Flüssigkeit, und einen gasförmigen Anteil umfasst und durch ein elektrisch leitendes Objekt, vorzugsweise ein Rohr oder eine Pipeline, strömt, gelöst, die Gegenstand des Anspruch 25 ist. Die Vorrichtung umfasst zumindest vier stromaufwärts entlang eines ersten Objektquerschnitts an oder nahe der Objektwand zu positionierende EMAT-Wandler sowie zumindest vier stromabwärts entlang eines zweiten Objektquerschnitts an oder nahe der Objektwand zu positionierende EMAT-Wandler. Jeweils zwei der stromaufwärts zu positionierten Wandler und jeweils zwei der stromabwärts zu positionierten Wandler sind auf dem Objekt gegenüberliegend, insbesondere im Fall eines rohrförmigen Objekts diametral, angeordnet. Die Positionen der stromaufwärts zu positionierten Wandler sind gegenüber der Positionen der stromabwärts zu positionierten Wandler nur in Längsrichtung des Objekts variiert. Weiterhin umfasst die Vorrichtung eine Steuereinheit, die, insbesondere anhand bestimmter Konfigurationsparameter, die für die jeweilige Messmethode notwendigen Wandler steuert, sowie eine Auswerteeinheit, die die aus den empfangenen Signalen erzeugten Daten auswertet.

Steuereinheit und Auswerteeinheit sind vorzugsweise in einer gemeinsamen Einheit zusammengefasst. Insbesondere sind Steuereinheit und Auswerteeinheit alternativ an unterschiedlichen Orten positioniert.

Mit einer derartigen Vorrichtung können mit einer relativ geringen Anzahl von EMAT-Wandlern mehrere Eigenschaften des Multiphasenstroms zuverlässig bestimmt werden. Durch das simple Setup von EMAT-Wandlern kann die Vorrichtung unter geringem Zeit- und Kostenaufwand hergestellt, installiert und gewartet werden. Vorzugsweise ist die Vorrichtung dabei zur Durchführung eines Verfahrens nach einem der Ansprüche 1 bis 24 geeignet und liefert insbesondere auch beim Vorliegen von Schwallfluss (slug flow) zuverlässige Ergebnisse.

Durch die Verwendung von EMAT-Wandlern kann auf eine aufwendige akustische Kopplung mittels insbesondere gelartiger Kopplungsmedien verzichtet werden. Zudem ergeben sich höhere Toleranzen bei der Positionierung der EMAT-Wandler zueinander, was die Herstellung und Installation der Vorrichtung vereinfacht.

In einer bevorzugten Ausführungsform der Erfindung decken entlang eines der Objektquerschnitte angeordneten EMAT-Wandler in Ihrer Gesamtheit das Objekt in Umfangsrichtung zumindest im Wesentlichen ab. Das Objekt gilt als im Wesentlichen abgedeckt, wenn der Umfang zu mindestens 90% von den Wandlern bedeckt ist. Das Objekt gilt insbesondere auch dann als vollständig bedeckt, wenn in Umfangrichtung des Objekts zwischen den einzelnen Wandlern technisch bedingte Mindestabstände vorhanden sind.

Gemäß einer weiteren bevorzugten Ausgestaltung der Erfindung umfasst die Vorrichtung entlang eines ersten Objektquerschnitts und/oder eines zweiten Objektquerschnitts jeweils zumindest sechs, vorzugsweise zumindest acht, insbesondere maximal 40, an oder nahe der Objektwand zu positionierende EMAT-Wandler. Insbesondere zur Bestimmung des Strömungsquerschnittanteils des gasförmigen Anteils des Multiphasenstroms basierend auf einer Auswertung von sich auf Basis von sich ausschließlich in der Objektwand ausbreitenden Teilen von stromabwärts oder stromaufwärts gesendeten Wellen ergebenden Signalen wird somit eine bessere Auflösung entlang des Objektumfangs erreicht, wodurch diese Eigenschaft zuverlässiger bestimmt werden kann. Die Anzahl der eingesetzten Wandler ist insbesondere abhängig vom Objekt- bzw. Rohrdurchmesser, wobei für größere Durchmesser tendenziell eine höhere Anzahl von Wandlern vorgesehen ist.

In einer weiteren bevorzugten Ausführungsform der Erfindung umfasst die Vorrichtung ferner zumindest einen das Objekt im Wesentlichen vollumfänglich umfassenden EMAT-Wandler. Ein derartiger Wandler kann eine Welle stromaufwärts oder stromabwärts vollumfänglich in die Objektwand senden, was insbesondere zur Bestimmung des Strömungsquerschnittanteils des gasförmigen Anteils des Multiphasenstroms von Vorteil ist. Dabei werden an zumindest zwei verschiedenen Umfangspositionen sich auf Basis des ausschließlich in der Objektwand ausbreitenden Teils dieser Welle ergebenden Signale empfangen. Diese gehen auf eine gemeinsame Welle oder ggf. auf einen gemeinsamen Wellenpuls bzw. Wellenburst zurück und lassen sich daher besser miteinander vergleichen. Das Objekt gilt als im Wesentlichen abgedeckt, wenn der Umfang zu mindestens 90%, bevorzugt zumindest 95%, von dem EMAT-Wandler bedeckt ist.

Gemäß einer weiteren bevorzugten Ausgestaltung der Erfindung umfasst die Vorrichtung zumindest zwei vollumfänglich ausgebildete EMAT-Wandler, wobei ein erster vollumfänglich ausgebildeter EMAT-Wandler stromaufwärts der zumindest vier stromaufwärts entlang eines ersten Objektquerschnitts an oder nahe der Objektwand zu positionierende EMAT-Wandler angeordnet ist und ein zweiter vollumfänglich ausgebildeter EMAT-Wandler stromabwärts der zumindest vier stromabwärts entlang eines zweiten Objektquerschnitts an oder nahe der Objektwand zu positionierende EMAT-Wandler angeordnet ist. Durch diese symmetrische Anordnung ist es möglich zumindest den Großteil der vorstehend beschriebenen Messverfahren unabhängig von der Hauptströmungsrichtung des Multiphasenstromes durchzuführen.

In einer weiteren bevorzugten Ausführungsform der Erfindung ist zumindest einer der Wandler als zumindest zwei zueinander räumlich versetze Spulen umfassender Phased-Array-Wandler ausgebildet. Durch den räumlichen Versatz in Kombination mit einem entsprechenden Phasenversatz der Anregungsströme kann dadurch eine Welle in der Objektwand gerichtet stromaufwärts oder stromabwärts gesendet werden. Die Aussendung von Wellen erfolgt somit zielgerichtet und insbesondere nach der jeweiligen Notwendigkeit der Messmethode. So können Wellen beispielsweise überwiegend in Richtung eines Empfängers gesendet werden. Entlang anderer Ausbreitungspfade werden hingegen keine oder kaum Wellen gesendet, wodurch auch ein besseres Signal-Rausch-Verhältnis erreicht wird.

Gemäß einer weiteren bevorzugten Ausgestaltung der Erfindung ist in radialer Richtung des Objekts über zumindest einem ersten Wandler, der zur Erzeugung von Ultraschallwellen mit einer ersten Wellenlänge ausgebildet ist, zumindest ein weiterer Wandler angeordnet, der zur Erzeugung von Ultraschallwellen mit einer anderen Wellenlänge ausgebildet ist. Dies ermöglicht je nach Anwendungsfall die Auswahl einer zur Bestimmung zumindest einer Eigenschaft optimalen Wellenlänge. Dabei sind vorzugsweise Wandler mit einer größeren Wellenlänge über Wandlern mit einer kleineren Wellenlänge angeordnet.

In einer weiteren bevorzugten Ausführungsform der Erfindung weist die Vorrichtung zumindest einen flexiblen Träger auf, in dem die stromaufwärts zu positionierenden Wandler und/oder die stromabwärts zu positionierenden Wandler und/oder die vollumfänglich ausgebildeten EMAT-Wandler angeordnet sind. Dadurch können die Wandler und insbesondere deren Spulen optimal an der Außenkontur der Objektwand positioniert werden. Zudem kann in Abhängigkeit vom Rohrdurchmesser eine Positionierung der Wandler zueinander (beispielsweise 12-, 3-, 6- und 9-Uhr-Position der nicht vollumfänglich ausgebildeten Wandler) vorgegeben werden, was die Installation der Vorrichtung praxistauglicher macht. Ferner ermöglicht dies die kostengünstige Herstellung von mehreren übereinander angeordneten Wandlern mit unterschiedlichen Wellenlängen. Vorzugsweise sind die Spulen der Wandler auf einer flexiblen Leiterplatte, insbesondere im Kupferbild, aufgedruckt.

In einer weiteren bevorzugten Ausführungsform der Erfindung sind die Spulen eines Wandlers in radialer Richtung einer der Längsmittelachse des Objekts übereinander, insbesondere in unterschiedlichen Schichten des Trägers, angeordnet. Dies ermöglicht eine besonders einfache Herstellung von insbesondere als Phased-Array-Wandlern mit zueinander versetzten Spulen ausgebildeten Wandlern.

Weitere Vorteile und Einzelheiten der Erfindung sind der nachfolgenden Figurenbeschreibung zu entnehmen.

In den schematisch dargestellten Figuren zeigen:

Fig. 1    ein erfindungsgemäßes Setup von EMAT-Wandlern,
Fig. 2    eine Prinzipdarstellung des Schwallflusses durch ein rohrförmiges Objekt,
Fig. 3    eine Ausführungsform der Erfindung,
Fig. 4    eine weitere Ausführungsform der Erfindung,
Fig. 5    eine weitere Ausführungsform der Erfindung,
Fig. 6    eine weitere Ausführungsform der Erfindung,
Fig. 7    eine weitere Ausführungsform der Erfindung,
Fig. 8    eine weitere Ausführungsform der Erfindung,
Fig. 9    eine weitere Ausführungsform der Erfindung,
Fig. 10    ein Diagramm mit Messergebnissen gemäß den Ausführungsformen der Fig. 8 und 9,
Fig. 11    eine weitere Ausführungsform der Erfindung,

Fig. 12    ein Diagramm mit Messergebnissen gemäß der Ausführungsform der Fig. 11,

Fig. 13    ein Wandler-Setup einer weiteren Ausführungsform der Erfindung,

Fig. 14    ein Diagramm mit Messergebnissen gemäß der Ausführungsform der Fig. 13,

Fig. 15    eine weitere Ausführungsform der Erfindung,

Fig. 16    eine weitere Ausführungsform der Erfindung,

Fig. 17    ein Detail eines Wandlers in einer weiteren Ausführungsform der Erfindung.

Gleich oder ähnlich wirkende Teile sind - sofern dienlich - mit identischen Bezugsziffern versehen. Einzelne technische Merkmale der nachfolgend beschriebenen Ausführungsbeispiele können auch mit den Merkmalen der vorbeschriebenen Ausführungsbeispiele, zumindest jedoch mit den Merkmalen eines der unabhängigen Ansprüche, zu erfindungsgemäßen Weiterbildungen führen.

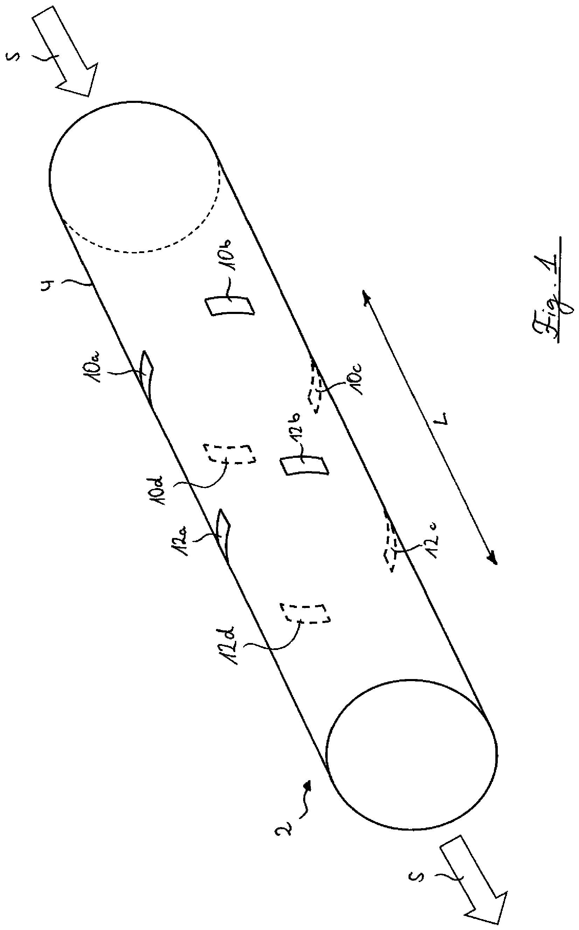

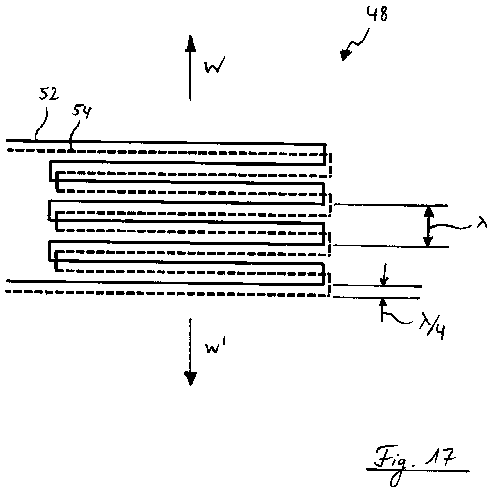

In Fig. 1 ist als Teil einer Vorrichtung zur nichtinvasiven Bestimmung von Eigenschaften eines Multiphasenstromes ein Wandler-Setup gezeigt, das an einem elektrisch leitenden, rohrförmigen Objekt 2 angeordnet ist, durch das der Multiphasenstrom (nicht gezeigt) entlang der Hauptströmungsrichtung S strömt. Die Vorrichtung weist dabei vier stromaufwärts entlang eines ersten Objektquerschnitts an oder nahe der Objektwand 4 positioniert EMAT-Wandler 10a, 10b, 10c, 10d auf, wobei je ein Wandler auf der 12-, 3-, 6- und 9-Uhr-Position angeordnet ist. Ferner weist die Vorrichtung vier stromabwärts entlang eines zweiten Objektquerschnitts an oder nahe der Objektwand 4 positionierte EMAT-Wandler 12a, 12b, 12c, 12d auf, wobei jeweils ein Wandler in einer 12-, 3-, 6- und 9-Uhr-Position angeordnet ist. Die Positionen der stromaufwärts positionierten Wandler 10a, 10b, 10c, 10d sind gegenüber den Positionen der stromabwärts positionierten Wandler 12a, 12b, 12c, 12d nur in Längsrichtung L des Objekts 2 variiert. Jeweils zwei der stromaufwärts positionierten Wandler 10a, 10b, 10c, 10d und jeweils zwei der stromabwärts positionierten Wandler 12a, 12b, 12c, 12d sind auf dem Objekt 2 diametral gegenüberliegend angeordnet. Die Wandler 10a, 10b, 10c, 10d, 12a, 12b, 12c, 12d sind vorzugsweise sowohl zum Senden als auch zum Empfangen ausgebildet. Insbesondere sind die Wandler 10a, 10b, 10c, 10d, 12a, 12b, 12c, 12d als Phased-Array-Wandler ausgebildet (s. Fig. 17).

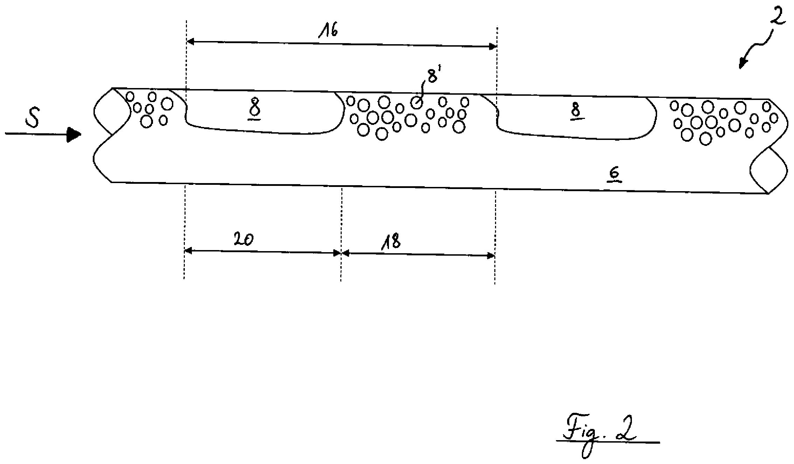

Fig. 2 zeigt eine schematische Darstellung des sogenannten Schwallflusses (slug flow) durch das Objekt 2. Der Multiphasenstrom umfasst dabei einen flüssigen Anteil 6 und einen gasförmigen Anteil, der überwiegend in Form großer Taylor-Blasen 8 und vereinzelt in Form von kleinen Gasbläschen 8' transportiert wird. Die Phasengrenzen zwischen dem gasförmigen Anteil und dem flüssigen Anteil 6 bilden Reflexionsquellen aus, an denen in den Multiphasenstrom eingekoppelte Wellen reflektiert werden können. Kleinere Gasbläschen 8' können nicht nur im Bereich zwischen zwei Taylor-Blasen 8, sondern in dem gesamten Volumen des flüssigen Anteils 6 auftreten. Ein Schwallabschnitt 18 und ein darauf folgender Taylor-Blasen-Abschnitt 20 bilden gemeinsam einen Schwallzyklus 16.

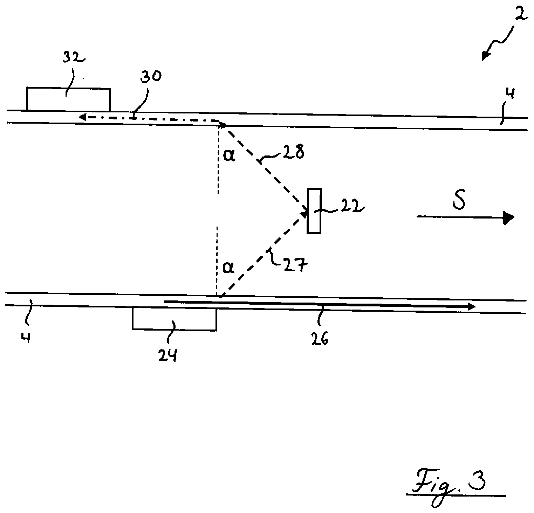

Die Fig. 3 bis 5 zeigen Ausführungsbeispiele, bei dem zur Bestimmung der Geschwindigkeit des flüssigen Anteils sich auf Basis einer von einer Reflexionsquelle 22 im Multiphasenstrom reflektierten Welle 28 ergebende Signale ausgewertet werden. Ein an oder nahe der Objektwand 4 auf einer ersten Seite des Objekts 2 angeordneter Sendewandler 24 erzeugt eine sich in der Objektwand 4 axial ausbreitende Welle 26, wobei ein Teil der Welle 26 unter einen Winkel in den Multiphasenstrom einkoppelt. Die in den Multiphasenstrom eingekoppelte Welle 27 trifft im Multiphasenstrom auf die Reflexionsquelle 22. Die an der Reflexionsfläche reflektierte Welle 28 koppelt unter dem Eintrittswinkel in die Objektwand 4 ein und erzeugt dort eine Welle 30 in der Objektwand 4. Ein sich auf Basis dieser in der Objektwand 4 erzeugten Welle 30 ergebendes Signal wird von einem Empfangswandler 32 empfangen. Insbesondere handelt es sich bei den Wellen 26 in der Objektwand 4 um Lamb-Wellen.

Fig. 3 zeigt eine Ausführungsform, bei der die Reflexionsquelle 22 eine senkrecht zur Hauptströmungsrichtung S des Multiphasenstroms verlaufende Reflexionsfläche aufweist. Die reflektierte Welle 28 koppelt auf der dem Sendewandler 24 gegenüberliegenden Seite in die Objektwand 4 ein, wo ein Empfangswandler 32 ein entsprechendes Signal empfängt.

Aus diesem Signal können Informationen bezüglich der Geschwindigkeit der Reflexionsquelle 22 gewonnen werden. Insbesondere kleine Reflexionsquellen 22 im flüssigen Anteil 6 des Multiphasenstroms, die beispielsweise auf kleinere Gasbläschen 8', Öltropfen im Wasser oder Wassertropfen im Öl zurückgehen, bewegen sich mit dem flüssigen Anteil 6 mit, so dass aus der Geschwindigkeit derartiger Reflexionsquellen 22 die Geschwindigkeit des flüssigen Anteils 6 ermittelt werden kann.

Vorzugsweise werden periodisch und für einen bestimmten Zeitraum Wellen(-pulse) erzeugt und sich die auf Basis von reflektierten Wellen 28 ergebenden Signale ausgewertet. Bewegt sich die Reflexionsquelle 22 in Richtung der Hauptströmungsrichtung S des Multiphasenstroms, erhöht sich das Zeitintervall zwischen dem Senden der Welle 26 und dem Empfangen des sich auf Basis der Welle 30 ergebenden Signals. Aus einer Verschiebung der zeitlichen Position eines auf eine bestimmte Reflexionsquelle 22 zurückgehenden Signals werden Rückschlüsse auf die axiale Geschwindigkeit der Reflexionsquelle 22 gezogen.

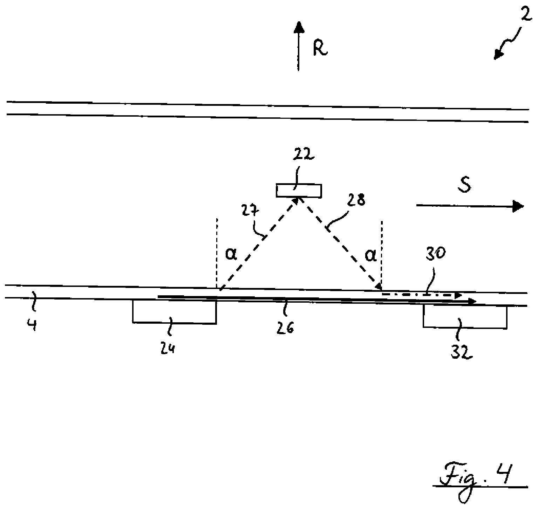

Fig. 4 zeigt ein Ausführungsbeispiel ähnlich Fig. 3 mit einer Reflexionsquelle 22, deren Reflexionsfläche parallel zur Hauptströmungsrichtung S des Multiphasenstroms verläuft. Ein entsprechendes Signal wird von einem Empfangswandler 32 empfangen, der auf derselben Seite angeordnet ist, wie der Sendewandler 24. Bewegt sich die Reflexionsquelle 22 senkrecht zur Hauptströmungsrichtung S des Multiphasenstroms bzw. in radialer Richtung R des rohrförmigen Objekts 2, können aus einer Verschiebung der zeitlichen Position des auf die Reflexionsquelle 22 zurückgehenden Signals Rückschlüsse auf die radiale Geschwindigkeit der Reflexionsquelle 22 gezogen werden.

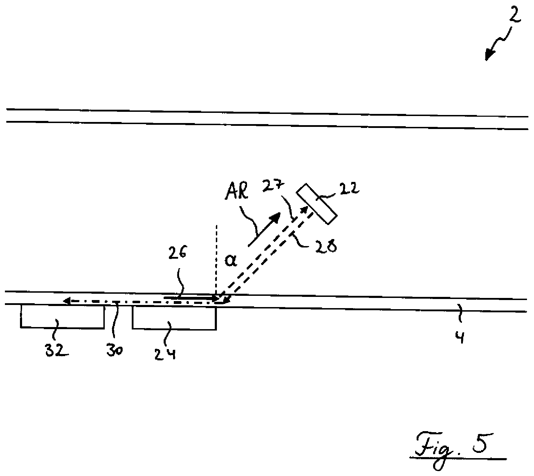

Fig. 5 zeigt ein Ausführungsbeispiel ähnlich Fig. 3 und Fig. 4 mit einer Reflexionsquelle 22, die eine senkrecht zur Hauptausbreitungsrichtung AR der in den Multiphasenstrom eingekoppelten Welle 27 verlaufenden Reflexionsfläche aufweist. Die reflektierte Welle 28 ist gegenüber der in den Multiphasenstrom gekoppelten Welle 27 um 180° zurückgeworfen. Die Geschwindigkeit dieser Reflexionsquelle 22 weist eine axiale sowie radiale Geschwindigkeitskomponente auf. Das sich aus der in die Objektwand 4 eingekoppelten Welle 30 ergebende Signal wird auf derselben Seite des Objekts 2 von einem Empfangswandler 32 empfangen. In einer weiteren Ausführungsform der Erfindung ist der Sendewandler 24 auch zum Empfangen des Signals ausgebildet.

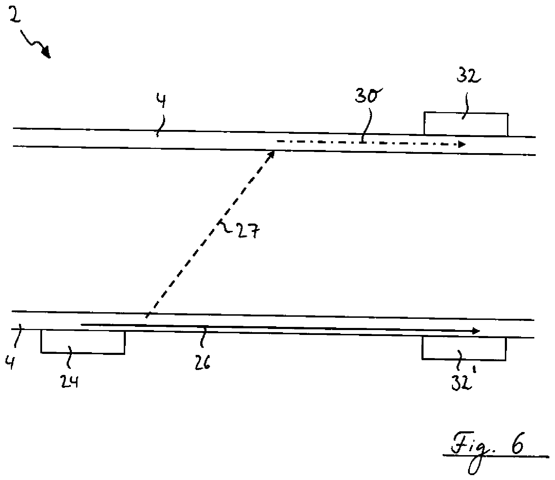

Fig. 6 zeigt schematisch den Ablauf einer Laufzeitmessung mittels einer zumindest in einem Teil des Multiphasenstroms eingekoppelten Welle 27. Ein an oder nahe der Objektwand 4 auf einer ersten Seite des Objekts 2 angeordneter Sendewandler 24 erzeugt eine sich in der Objektwand 4 axial ausbreitende Welle 26. Ein Teil dieser Welle 26 koppelt in den Multiphasenstrom ein. Die in zumindest einen Teil des Multiphasenstroms eingekoppelte Welle 27 quert den Multiphasenstrom und koppelt auf der gegenüberliegenden Seite in die Objektwand 4 ein und erzeugt dort eine Welle 30 in der Objektwand 4. Aus dem Zeitintervall zwischen dem Senden der Welle 26 und dem Empfangen des sich auf Basis der Welle 30 ergebenden Signals durch einen auf der gegenüberliegenden Seite angeordneten Empfangswandler 32 wird in einer Ausführungsform der Erfindung die Schallgeschwindigkeit des Mediums ermittelt. Vorzugsweise wird dabei die Schallgeschwindigkeit des flüssigen Anteils 6 ermittelt. Unter Kenntnis der Schallgeschwindigkeiten der einzelnen Komponenten des flüssigen Anteils 6, d. h. des Wasser und/oder der kohlenwasserstoffhaltigen Flüssigkeit, kann der jeweilige Anteil der Komponente und somit der Wassergehalt im flüssigen Anteil selbst ermittelt werden.

Zur Berechnung der Laufzeit wird ein sich auf Basis des sich ausschließlich in der Objektwand 4 ausbreitenden Teils der gesendeten Welle 26 ergebendes Signal (direktes Wandsignal) als Referenz verwendet. Dieses wird von einem weiteren Empfangswandler 32' auf derselben Seite, an der der Sendewandler 24 angeordnet ist, empfangen.

Vorzugsweise weist die den Multiphasenstrom querende Welle 27 einen sich zwischen einer 3-Uhr-Position und einer 9-Uhr-Position erstreckenden Ausbreitungspfad auf. Dieser verläuft insbesondere in einer im Wesentlichen quer zur Richtung der Gravitation verlaufenden Ebene. Dadurch wird die Wahrscheinlichkeit erhöht, dass die Welle 27 den flüssigen Anteil 6 quert, da sich bei den meisten Strömungsarten der überwiegende Teil des gasförmigen Anteils in einem oberen Bereich des Objekts 2 (10-Uhr-Position bis 2-Uhr-Position) sammelt.

Aus der Durchführung einer Laufzeitmessung gemäß Fig. 6 sowohl stromabwärts als auch stromaufwärts wird gemäß einer weiteren Ausführungsform der Erfindung eine Laufzeitdifferenz der stromaufwärts und stromabwärts gemessenen Signale ermittelt, aus der die Geschwindigkeit des flüssigen Anteils bestimmt wird. Dies wird in einer weiteren erfindungsgemäßen Ausführung durch eine Anordnung von zwei stromaufwärts auf gegenüberliegenden Seiten des Objekts angeordneten Wandlern 10b, 10d und zwei stromabwärts auf zwei gegenüberliegenden Seiten des Objekts angeordneten Wandlern 12b, 12d realisiert.

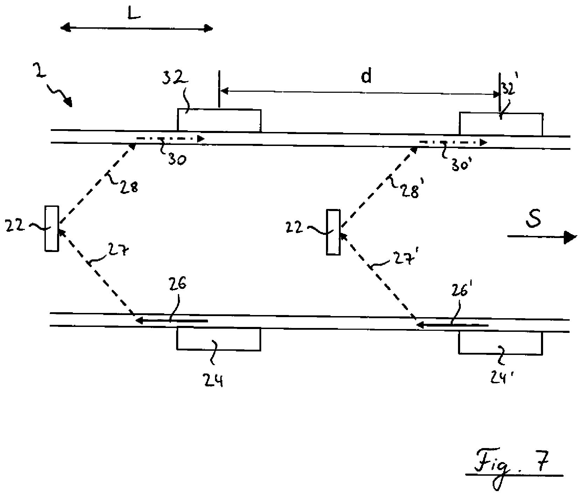

Fig. 7 zeigt ein erfindungsgemäßes Ausführungsbeispiel, bei dem zur Bestimmung der Geschwindigkeit des gasförmigen Anteils zwei Signale räumlich miteinander korreliert werden. Entlang eines ersten Objektquerschnitts sind einander gegenüberliegend ein Sendewandler 24 und ein Empfangswandler 32 angeordnet. Entlang eines zweiten Objektquerschnitts, der in Längsrichtung L des Objekts 2 um den Abstand d vom ersten Objektquerschnitt beabstandet ist, sind ein weiterer Sendewandler 24' sowie ein weiterer Empfangswandler 32' angeordnet. Beispielsweise wird ein Signal, das sich auf Basis einer von einer an einer Reflexionsquelle 22 reflektierten Welle 28 ergibt, an dem ersten Objektquerschnitt beobachtet. Nach einer gewissen Zeit wird dasselbe bzw. ein ähnliches Signal an dem zweiten Objektquerschnitt beobachtet. Aus dem Abstand d und der zwischen den Beobachtungen vergangenen Zeit kann die Geschwindigkeit der Reflexionsquelle 22 bestimmt werden.

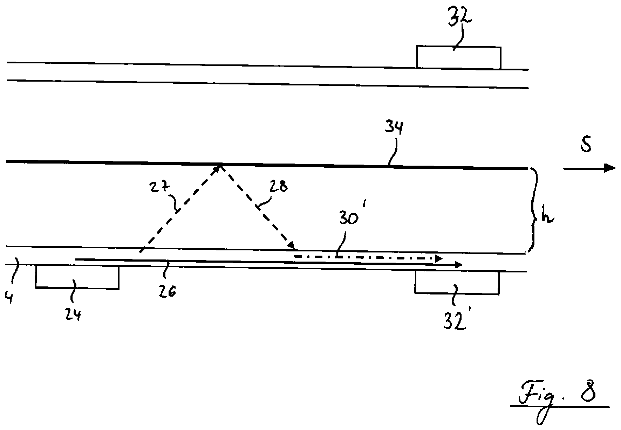

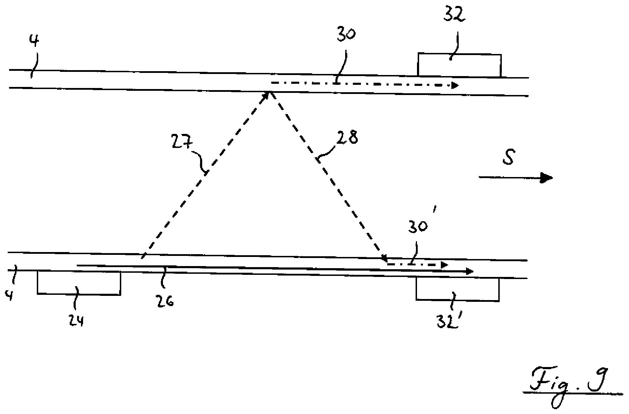

Anhand der Figuren 8 und 9 wird die Bestimmung des Strömungsquerschnittsanteils des gasförmigen Anteils auf Basis einer stromabwärts oder stromaufwärts gesendeten, zumindest in einen Teil des Multiphasenstroms eingekoppelten Welle 27 erläutert. Ein in einer untersten Position (6-Uhr-Position) an oder nahe der Objektwand 4 angeordneter Sendewandler 24 erzeugt eine sich in der Objektwand 4 axial ausbreitende Welle 26, wobei ein Teil der Welle unter einem bestimmten Winkel im Multiphasenstrom einkoppelt. In Abhängigkeit vom Strömungsquerschnittsanteil des gasförmigen Anteils wird die in den Multiphasenstrom eingekoppelte Welle 27 entweder an einer parallel zur Hauptströmungsrichtung S des Multiphasenstroms verlaufenden horizontalen Phasengrenze 34 reflektiert (Fig. 8) oder quert den Multiphasenstrom zumindest einmal (Fig. 9). Der Strömungsquerschnittsanteil des gasförmigen Anteils wird in einer erfindungsgemäßen Ausführungsform über die Höhe h der horizontalen Phasengrenze 34 bestimmt. Die Höhe h wird insbesondere aus der Laufzeit eines sich auf Basis der an der Reflexionsfläche reflektierten Welle 28 ergebenden Signals, das von einem an einer untersten Position (6-Uhr-Position) angeordneten Empfangswandler 32' empfangen wird, berechnet. Auf diese Art und Weise werden vorzugsweise größere Gasblasen im Multiphasenstrom, insbesondere Taylor-Blasen 8 bei Vorliegen von Schwallfluss, detektiert. Insbesondere wird zur Berechnung der Laufzeit ein sich auf Basis eines ausschließlich in der Objektwand ausbreitenden Teils der gesendeten Welle 26 ergebendes Signal (direktes Wandsignal) als Referenz verwendet.

Für den Fall, dass der Multiphasenstrom entlang zumindest eines Ausbreitungspfades der in den Multiphasenstrom eingekoppelten Welle 27 im Wesentlichen (zumindest bis auf kleine Gasbläschen 8') aus flüssigem Anteil 6 besteht, kann die Welle 27 den Multiphasenstrom queren und auf der gegenüberliegenden Seite der Objektwand 4 einkoppeln (Fig. 9). In diesem Fall wird ein sich auf Basis der den Multiphasenstrom querenden Welle 27 ergebendes Signal von einem an einer obersten Position (12-Uhr-Position) an oder nahe der Objektwand 4 angeordneten Empfangswandler 32 empfangen. Für den Fall, dass der Multiphasenstrom entlang eines größeren Längsabschnitts des Objekts 2 aus flüssigem Anteil 6 besteht, kann die von einer ersten Seite der Objektwand 4 in den Multiphasenstrom eingekoppelte Welle 27 diesen zweimal queren, wobei die Welle von der gegenüberliegenden Seite der Objektwand 4 reflektiert wird (reflektierte Welle 28) und auf der ersten Seite der Objektwand 4 in diese einkoppelt, wo sie eine Welle 30' in der Objektwand 4 erzeugt. Ein sich aus dieser Welle 30' ergebendes Signal wird von dem Empfangswandler 32' empfangen.

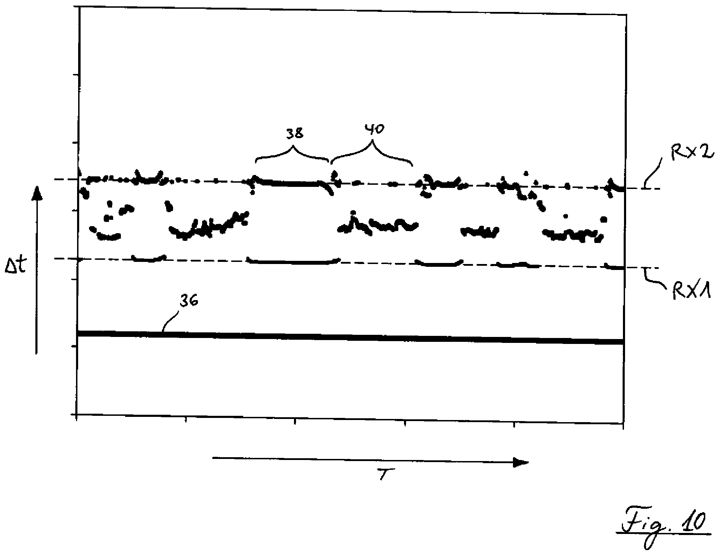

In Fig. 10 sind die von den Empfangswandlern 32, 32' über einen längeren Zeitraum wiederholt aufgezeichneten Signale entsprechend ihrer Laufzeit $\Delta t$ dargestellt. Der an der untersten Position (6-Uhr-Position) angeordnete Empfangswandler 32' empfängt zum einen das direkte Wandsignal 36, das insbesondere als Referenz zur Berechnung der Laufzeiten $\Delta t$ verwendet wird. Der an der obersten Position (12-Uhr-Position) angeordnete Empfangswandler 32 empfängt ausschließlich dann ein Signal, wenn entlang eines Ausbreitungspfades der in den Multiphasenstrom eingekoppelten Welle 27 der Strömungsquerschnitt vollständig vom flüssigen Anteil 6 des Multiphasenstroms eingenommen ist. Diese Signale sind entlang der Linie RX1 dargestellt. Kann die in den Multiphasenstrom eingekoppelte Welle 27 diesen zweimal queren, empfängt auch der an der 6-Uhr-Position angeordnete Empfangswandler 32' ein entsprechendes Signal, das sich insbesondere durch die maximal mögliche Laufzeit $\Delta t$ kennzeichnet. Derartige Signale sind entlang der Linie RX2 dargestellt. An der horizontalen Phasengrenze 34 reflektierte Signale, die von dem an der 6-Uhr-Position angeordneten Empfangswandler 32' empfangen werden, befinden sich zwischen den Linien RX1 und RX2. Es ist erkennbar, dass sich über die Zeit T Transmissionsbereiche 38, die insbesondere auf einen Schwallabschnitt 18 hindeuten, und Reflexionsbereiche 40, die insbesondere auf einen Taylor-Blasen-Abschnitt 20 hindeuten, zyklisch aufeinander folgen. Die gemessenen Laufzeiten $\Delta t$ zu einer bestimmten Zeit T geben Aufschluss über die Höhe der horizontalen Phasengrenze und damit über den Strömungsquerschnittanteil des gasförmigen Anteils.

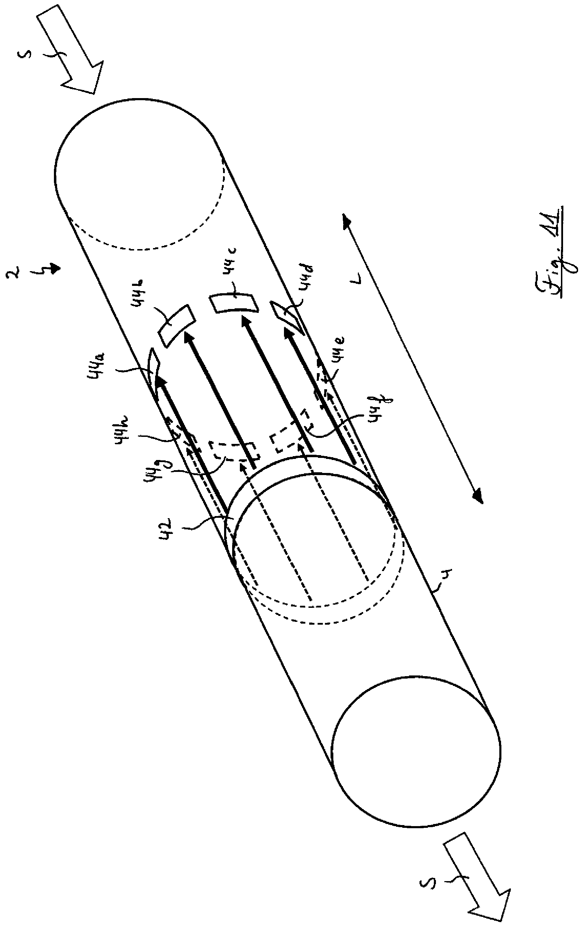

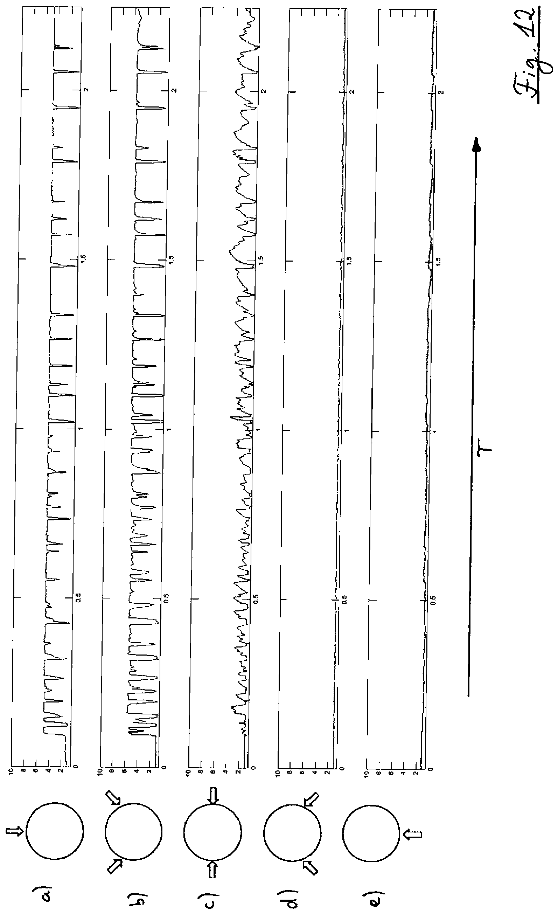

Eine weitere Methode zur Bestimmung des Strömungsquerschnittsanteils des gasförmigen Anteils auf Basis eines sich ausschließlich in der Objektwand ausbreitenden Teils einer stromabwärts oder stromaufwärts gesendeten Welle wird im Folgenden anhand der Fig. 11 und 12 beschrieben.

Fig. 11 zeigt ein rohrförmiges Objekt mit einem entlang eines ersten Objektquerschnitts an oder nahe der Objektwand 4 angeordneten vollumfänglichen Sendewandler 42, der stromaufwärts eine Welle vollumfänglich in die Objektwand sendet, wobei die sich auf Basis des ausschließlich in der Objektwand 4 ausbreitenden Teils dieser Welle ergebenden Signale an einem von dem ersten Objektquerschnitt in Längsrichtung L des Objekts 2 beabstandeten zweiten Objektquerschnitt an verschiedenen Umfangspositionen von acht entlang des Umfangs angeordneten Empfangswandlern 44a bis 44h empfangen werden.

Die in der Objektwand 4 erzeugte Welle koppelt je nach Zusammensetzung des an die Objektwand 4 angrenzenden Teils des Multiphasenstroms, insbesondere abhängig von der Dichte in diesem Teil, unterschiedlich gut in den Multiphasenstrom ein. Wie aus Fig. 12 deutlich wird, lassen sich aus der Abschwächung der Amplitude Rückschlüsse auf die Zusammensetzung des an die Objektwand 4 angrenzenden Teils des Multiphasenstroms ziehen. Fig. 12 zeigt den Verlauf der Amplituden über die Zeit T von sich auf Basis des ausschließlich in der Objektwand 4 ausbreitenden Teils der gesendeten Welle ergebenden Signalen, die an verschiedenen Umfangspositionen des Objekts empfangen wurden. Diagramm e) zeigt die Auswertung der auf einer 6-Uhr-Position empfangenen Signale. Die Amplitude des direkten Signals ist relativ klein, da ein Großteil der Welle in den sich hinter dem Teil der Objektwand 4 befindenden Teil des Multiphasenstroms eingekoppelt ist. Daraus kann geschlossen werden, dass der sich hinter dem Teil der Objektwand 4 befindende Teil des Multiphasenstroms eine relativ hohe Dichte aufweist, also insbesondere zum flüssigen Anteil 6 des Multiphasenstroms gehört. Gleiches gilt für das Diagramm d), bei dem ein ähnliches Signal auf der 4-Uhr- und der 8-Uhr-Position gemessen wurde.

Demgegenüber sind im Diagramm a) auf der 12-Uhr-Position empfangene Signale ausgewertet. Die Amplitude verharrt über weitere Teile auf einem Plateau. Dies deutet darauf hin, dass die überwiegende Zeit hinter dem entsprechenden Teil der Objektwand 4 ein Teil des Multiphasenstroms mit einer geringeren Dichte, insbesondere der gasförmige Anteil, vorhanden ist. Die wiederkehrenden Täler bzw. Amplitudeneinbrüche sind darauf zurückzuführen, dass zu diesen Zeitpunkten der Strömungsquerschnitt von einem Teil des Multiphasenstroms mit einer relativ hohen Dichte, insbesondere dem flüssigen Anteil 6, eingenommen wird. Ähnliches ist beim den Diagramm b) (2-Uhr- und 10-Uhr-Position) zu beobachten. Beim Diagramm c), wo auf einer 3-Uhr- und 6-Uhr-Position empfangene Signale ausgewertet wurden, schwankt die Amplitude am meisten, was darauf hindeutet, dass hinter den entsprechenden Teilen der Objektwand 4 flüssiger und gasförmiger Anteil abwechselnd vorkommen.

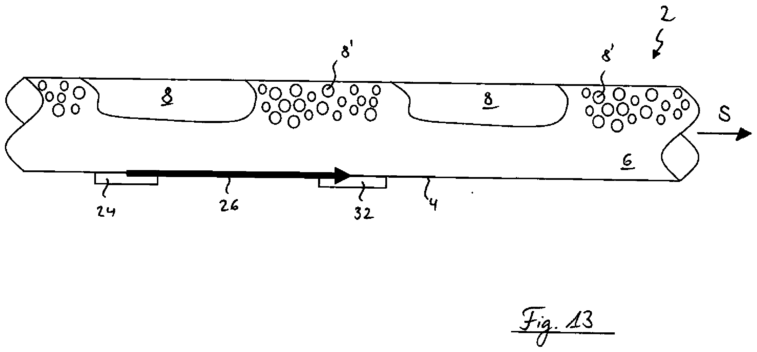

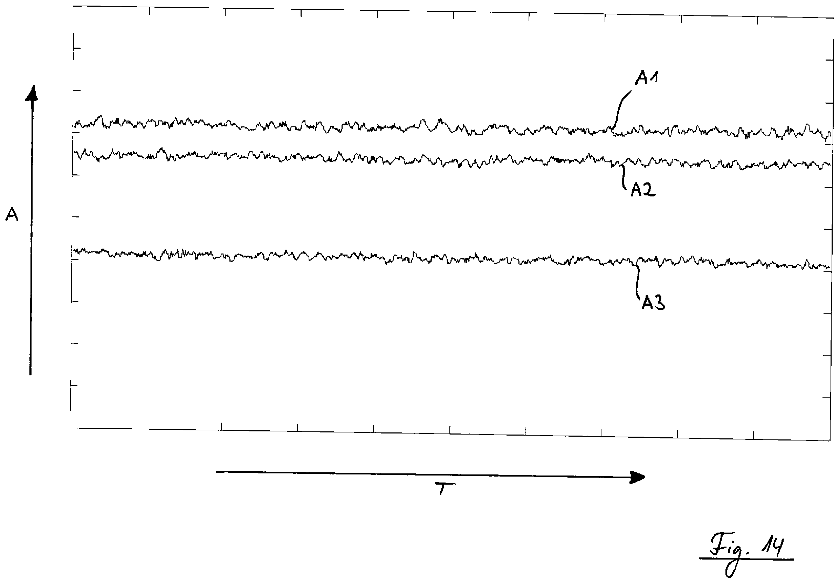

Gemäß der erfindungsgemäßen Ausführungsform nach Fig. 13 wird ein sich ausschließlich in der Objektwand 4 ausbreitender Teil einer von einem in einer 6-Uhr-Position angeordneten Sendewandler 24 gesendeten Welle ergebendes Signal zur Bestimmung des Wassergehalts im flüssigen Anteil 6 des Multiphasenstroms gezeigt. Das von dem Empfangswandler 32 empfangene Signal ist entsprechend seiner Amplitude in dem in Fig. 14 gezeigten Diagramm über die Zeit aufgetragen. Die Messkurve A1 entspricht dabei einem Wasseranteil von 0%, die Messkurve A2 einen Wasseranteil von 20% und die Messkurve A3 einem Wasseranteil von 100%. Der Anteil der Flussrate des gasförmigen Anteils an der Gesamtflussphase lag bei allen drei Messungen bei 33%.

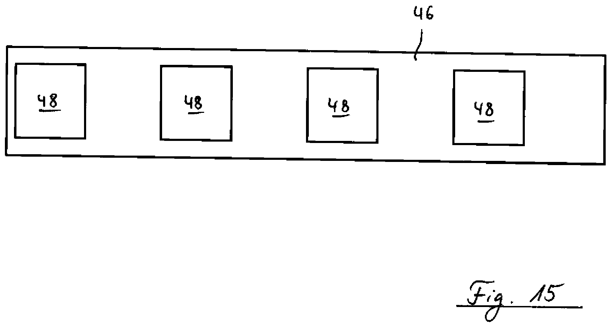

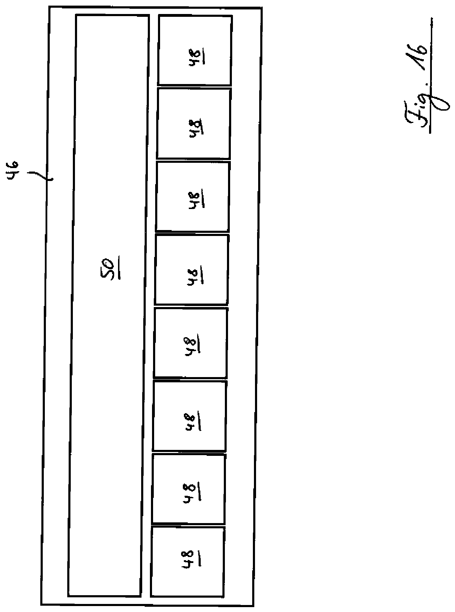

Fig. 15 zeigt einen flexiblen Träger 46, der insbesondere als gedruckte Platine ausgebildet ist, mit vier entlang eines Objektquerschnitts anzuordnenden Wandlern 48. Die Wandler 48 können somit optimal an der Außenkontur der Objektwand 4 positioniert werden, wobei die Positionierung der Wandler zueinander (12-Uhr-, 3-Uhr-, 6-Uhr- und 9-Uhr-Position) vorgegeben ist, was die Installation der Vorrichtung praxistauglicher macht. In einer weiteren erfindungsgemäßen Ausführung gemäß Fig. 16 sind in einem flexiblen Träger 46, der insbesondere als gedruckte Leiterplatte ausgebildet ist, neben einem das Objekt 2 im Wesentlichen vollumfänglich umfassenden Wandler 50 zudem acht in ihrer Gesamtheit das Objekt 2 in Umfangsrichtung im Wesentlichen abdeckende Wandler 48 angeordnet.

Fig. 17 zeigt schematisch einen Aufbau eines als Phased-Array-Wandler ausgebildeten Wandlers 48 umfassend zwei zueinander versetzte Spulen 52, 54, die zueinander um $\lambda/4$ räumlich versetzt sind, wobei $\lambda$ die Wellenlänge der in der Objektwand 4 erzeugten Welle 26 ist. Durch den räumlichen Versatz in Kombination mit einem entsprechenden Phasenversatz der Anregungsströme von $\pm 90°$ kann die Welle 26 in der Objektwand 4 (nicht gezeigt) - je nach Vorzeichen des Phasenversatzes - gerichtet in Richtung W oder in Richtung W' gesendet werden.

**Patentansprüche**

1. Verfahren zur nichtinvasiven Bestimmung von Eigenschaften eines Multiphasenstromes, der einen flüssigen Anteil (6), insbesondere umfassend Wasser und/oder eine kohlenwasserstoffhaltige Flüssigkeit, und einen gasförmigen Anteil umfasst und durch ein elektrisch leitendes Objekt (2), vorzugsweise ein Rohr oder eine Pipeline, strömt, wobei die Eigenschaften des Multiphasenstromes insbesondere zumindest die Geschwindigkeit des gasförmigen Anteils, die Geschwindigkeit des flüssigen Anteils (6), den Strömungsquerschnittanteil des gasförmigen Anteils und/oder den Wassergehalt im flüssigen Anteil (6) umfassen, wobei bei dem Verfahren

   - unter Verwendung eines einzigen Setups mit einer Mehrzahl von EMAT-Wandlern (10a, 10b, 10c, 10d, 12a, 12b, 12c, 12d, 24, 24', 32, 32', 42, 44a, 44b, 44c, 44d, 44e, 44f, 44g, 44h, 48, 50), insbesondere nach Vorrichtungsanspruch 24,
   - zur Bestimmung der Geschwindigkeit des gasförmigen Anteils zumindest zwei Signale räumlich miteinander korreliert werden,
   - zur Bestimmung der Geschwindigkeit des flüssigen Anteils (6) zumindest ein sich auf Basis einer von einer Reflexionsquelle (22) im Multiphasenstrom reflektierten Welle (28) ergebendes Signal ausgewertet wird und/oder zumindest ein sich auf Basis einer stromaufwärts gesendeten, zumindest in einen Teil des Multiphasenstroms eingekoppelten Welle (27) ergebendes Signal und zumindest ein sich auf Basis einer stromabwärts gesendeten, zumindest in einen Teil des Multiphasenstroms eingekoppelten Welle (27) ergebendes Signal ausgewertet wird und
   - zur Bestimmung des Strömungsquerschnittanteils des gasförmigen Anteils zumindest ein sich auf Basis einer stromabwärts oder stromaufwärts gesendeten, zumindest in einen Teil des Multiphasenstroms eingekoppelten Welle (27) ergebendes Signal ausgewertet wird und/oder zumindest ein sich auf Basis eines sich ausschließlich in der Objektwand (4) ausbreitenden Teils einer stromabwärts oder stromaufwärts gesendeten Welle (26) ergebendes Signal ausgewertet wird.

2. Verfahren nach Anspruch 1, **dadurch gekennzeichnet, dass** aus zumindest zwei Eigenschaften des Multiphasenstroms zumindest die Flussrate des flüssigen Anteils (6) und/oder die Flussrate des gasförmigen Anteils ermittelt werden.

3. Verfahren nach einem der vorherigen Ansprüche, **dadurch gekennzeichnet, dass** zur Bestimmung des Wassergehalts im flüssigen Anteil (6) zumindest ein sich auf Basis eines sich ausschließlich in der Objektwand (4) ausbreitenden Teils einer stromabwärts oder stromaufwärts gesendeten Welle (26) ergebendes Signal und/oder zumindest ein sich auf Basis einer stromaufwärts oder stromabwärts gesendeten, zumindest in einen Teil des Multiphasenstroms eingekoppelten Welle (27) ergebendes Signal ausgewertet wird.

4. Verfahren nach Anspruch 3, **dadurch gekennzeichnet, dass** zur Bestimmung des Wassergehalts im flüssigen Anteil (6) zumindest ein sich auf Basis einer stromaufwärts oder stromabwärts gesendeten, zumindest in einen Teil des Multiphasenstroms eingekoppelten Welle (27) ergebendes Signal und ein sich auf Basis einer weiteren in die jeweils andere Richtung (stromaufwärts oder stromabwärts) gesendeten, zumindest in einen Teil des Multiphasenstroms eingekoppelten Welle (27) ergebendes Signal ausgewertet wird.

5. Verfahren nach einem der vorherigen Ansprüche, **dadurch gekennzeichnet, dass**, insbesondere zur Bestimmung des Strömungsquerschnittanteils des gasförmigen Anteils und/oder zur Bestimmung des Wassergehalts im flüssigen Anteil (6), das zumindest eine sich auf Basis eines sich ausschließlich in der Objektwand (4) ausbreitenden Teils einer an einer ersten Position stromaufwärts oder stromabwärts gesendeten Welle (26) ergebende Signal an einer in Längsrichtung (L) des Objekts (2) von der ersten Position beabstandeten zweiten Position empfangen wird, wobei anhand der Amplitude (A) des Signals die Zusammensetzung des an die Objektwand (4) angrenzenden Teils des Multiphasenstromes ermittelt wird.

6. Verfahren nach einem der vorherigen Ansprüche, **dadurch gekennzeichnet, dass** bei einem sich horizontal erstreckenden Objekt (2) zumindest ein Teil der stromaufwärts und/oder stromabwärts gesendeten, zumindest in einen Teil des Multiphasenstroms eingekoppelten Wellen (27, 28) jeweils zumindest einen sich zwischen einer 3-Uhr-Position und einer 9-Uhr-Position erstreckenden Ausbreitungspfad aufweist.

7. Verfahren nach einem der vorherigen Ansprüche, **dadurch gekennzeichnet, dass**, insbesondere zur Bestimmung der Geschwindigkeit des gasförmigen Anteils, zumindest zwei Signale an zwei in Längsrichtung (L) des Objekts (2) voneinander beabstandeten Positionen miteinander korreliert werden.

8. Verfahren nach einem der vorherigen Ansprüche, **dadurch gekennzeichnet, dass**, insbesondere zur Bestimmung der Geschwindigkeit des flüssigen Anteils (6) und/oder der Geschwindigkeit des gasförmigen Anteils, eine Frequenzverschiebung des sich auf Basis einer von einer Reflexionsquelle im Multiphasenstrom reflektierten Welle (28) ergebenden Signals ausgewertet wird.

9. Verfahren nach einem der vorherigen Ansprüche, **dadurch gekennzeichnet, dass**, insbesondere zur Bestimmung der Geschwindigkeit des flüssigen Anteils (6), periodisch Wellen (26) erzeugt und die sich auf Basis von reflektierten Wellen (28) ergebenden Signale ausgewertet werden, wobei aus einer Verschiebung der zeitlichen Position zumindest eines auf eine bestimmte Reflexionsquelle (22) zurückgehenden Signals zumindest die Geschwindigkeit der Reflexionsquelle (22) ermittelt wird.

10. Verfahren nach einem der vorherigen Ansprüche, **dadurch gekennzeichnet, dass**, insbesondere zur Bestimmung der Geschwindigkeit des flüssigen Anteils (6), zumindest ein sich auf Basis einer von einer Reflexionsfläche, die senkrecht oder parallel zur Hauptströmungsrichtung (S) des Multiphasenstroms oder senkrecht zur Hauptausbreitungsrichtung (AR) der in den Multiphasenstrom eingekoppelten Welle (27) verläuft, reflektierten Welle (28) ergebendes Signal ausgewertet wird.

11. Verfahren nach einem der vorherigen Ansprüche, **dadurch gekennzeichnet, dass**, insbesondere zur Bestimmung der Geschwindigkeit des flüssigen Anteils (6), die Laufzeitdifferenz zwischen dem zumindest einen sich auf Basis der stromaufwärts gesendeten, in zumindest einen Teil des Multiphasenstroms eingekoppelten Welle (27) ergebenden Signal und dem zumindest einen sich auf Basis der stromabwärts gesendeten, in zumindest einen Teil des Multiphasenstroms eingekoppelten Welle (27) ergebenden Signal ausgewertet wird.

12. Verfahren nach einem der vorherigen Ansprüche, **dadurch gekennzeichnet, dass**, insbesondere zur Bestimmung des Strömungsquerschnittanteils des gasförmigen Anteils, zumindest eine sich auf Basis einer von einer Reflexionsquelle (22) im Multiphasenstrom reflektierte Welle (28), die sich zumindest teilweise aus der an einer ersten Position stromabwärts oder stromaufwärts gesendeten, in zumindest einen Teil des Multiphasenstroms eingekoppelten Welle (27) ergibt, ergebende Signal an einer in Längsrichtung (L) des Objekts (2) von der ersten Position beabstandeten zweiten Position empfangen wird.

13. Verfahren nach Anspruch 12, **dadurch gekennzeichnet, dass** die an einer ersten Position stromabwärts oder stromaufwärts gesendete Welle (26) von einer tiefsten Umfangsposition (6-Uhr-Position) gesendet wird und das sich auf Basis der von einer Reflexionsquelle (22) im Multiphasenstrom reflektierten Welle (28) ergebende Signal an einer tiefsten Umfangsposition (6-Uhr-Position) empfangen wird.

14. Verfahren nach einem der vorherigen Ansprüche, **dadurch gekennzeichnet, dass**, insbesondere zur Bestimmung des Strömungsquerschnittanteils des gasförmigen Anteils, zumindest ein sich auf Basis einer durch zumindest einen Teil des Multiphasenstroms transmittierten Welle (27) ergebendes Signal ausgewertet wird, wobei sich die transmittierte Welle (27) zumindest teilweise aus der stromabwärts oder stromaufwärts in den Multiphasenstrom gesendeten Welle (26) ergibt.

15. Verfahren nach einem der vorherigen Ansprüche, **dadurch gekennzeichnet, dass**, insbesondere zur Bestimmung des Strömungsquerschnittanteils des gasförmigen Anteils, zumindest ein sich aus einer zweimal durch zumindest einen Teil des Multiphasenstroms transmittierten Welle (28) ergebendes Signal ausgewertet wird, wobei sich die zweimal transmittierte Welle (28) zumindest teilweise aus der stromabwärts oder stromaufwärts in den Multiphasenstrom gesendeten Welle (26) ergibt.

16. Verfahren nach einem der vorherigen Ansprüche, **dadurch gekennzeichnet, dass**, insbesondere zur Bestimmung des Strömungsquerschnittanteils des gasförmigen Anteils, die zumindest eine stromaufwärts oder stromabwärts gesendete Welle (26) entlang eines ersten Objektquerschnitts vollumfänglich in die Objektwand (4) gesendet wird und die sich auf Basis des ausschließlich in der Objektwand (4) ausbreitenden Teils dieser Welle (26) ergebenden Signale an einem von dem ersten Objektquerschnitt in Längsrichtung (L) des Objekts (2) beabstandeten zweiten Objektquerschnitt an zumindest zwei verschiedenen Umfangspositionen empfangen werden.

17. Verfahren nach einem der vorherigen Ansprüche, **dadurch gekennzeichnet, dass** zumindest einer der Messmethoden bestimmte Konfigurationsparameter zugeordnet sind, anhand dessen die für die Messmethode notwendigen Wandler (10a, 10b, 10c, 10d, 12a, 12b, 12c, 12d, 24, 24', 32, 32', 42, 44a, 44b, 44c, 44d, 44e, 44f, 44g, 44h, 48, 50) gesteuert werden.

18. Verfahren nach einem der vorherigen Ansprüche, **dadurch gekennzeichnet, dass** zumindest einer der Wandler (10a, 10b, 10c, 10d, 12a, 12b, 12c, 12d, 24, 24', 32, 32', 42, 44a, 44b, 44c, 44d, 44e, 44f, 44g, 44h, 48, 50) sowohl als Sender als auch als Empfänger eingesetzt wird.

19. Verfahren nach einem der vorherigen Ansprüche, **dadurch gekennzeichnet, dass** zumindest einer der Wandler (10a, 10b, 10c, 10d, 12a, 12b, 12c, 12d, 24, 24', 32, 32', 42, 44a, 44b, 44c, 44d, 44e, 44f, 44g, 44h, 48, 50) gerichtet stromaufwärts und/oder stromabwärts sendet.

20. Verfahren nach einem der vorherigen Ansprüche, **dadurch gekennzeichnet, dass** zumindest einer der Wandler (10a, 10b, 10c, 10d, 12a, 12b, 12c, 12d, 24, 24', 32, 32', 42, 44a, 44b, 44c, 44d, 44e, 44f, 44g, 44h, 48, 50) Lamb-Wellen in der Objektwand (4) erzeugt.

21. Verfahren nach einem der vorherigen Ansprüche, **dadurch gekennzeichnet, dass** für zumindest eine Eigenschaft des Multiphasenstromes die diese bestimmende Messmethode in Abhängigkeit des Verhältnisses von gasförmigem Anteil zu flüssigem Anteil (6) des Multiphasenstromes, insbesondere in Abhängigkeit der Flussrate des flüssigen Anteils (6) und/oder der Flussrate des gasförmigen Anteils, ausgewählt wird.

22. Verfahren nach einem der vorherigen Ansprüche, **dadurch gekennzeichnet, dass** zur Bestimmung zumindest einer der Eigenschaften für zumindest eine Messmethode eine Mehrzahl von Einzelmessungen durchgeführt und ausgewertet wird, wobei insbesondere vorzugsweise ein Mittelwert und/oder ein Maximum ermittelt wird.

23. Verfahren nach einem der vorherigen Ansprüche, **dadurch gekennzeichnet, dass** für zumindest eine Messmethode eine Pulswiederholungsfrequenz von zumindest 200 Hz, bevorzugt zumindest 400 Hz, besonders bevorzugt zumindest 800 Hz, und maximal 5 kHz verwendet wird.

24. Vorrichtung zur nichtinvasiven Bestimmung von Eigenschaften eines Multiphasenstromes, der einen flüssigen Anteil (6), insbesondere umfassend Wasser und/oder eine kohlenwasserstoffhaltige Flüssigkeit, und einen gasförmigen Anteil umfasst und durch ein elektrisch leitendes Objekt (2), vorzugsweise ein Rohr oder eine Pipeline, strömt, umfassend

- zumindest vier stromaufwärts entlang eines ersten Objektquerschnitts an oder nahe der Objektwand (4) zu positionierende EMAT-Wandler (10a, 10b, 10c, 10d, 24, 24', 32, 32', 48) sowie
- zumindest vier stromabwärts entlang eines zweiten Objektquerschnitts an oder nahe der Objektwand (4) zu positionierende EMAT-Wandler (12a, 12b, 12c, 12d, 24, 24', 32, 32', 48),
- wobei jeweils zwei der stromaufwärts zu positionierenden Wandler (10a, 10b, 10c, 10d, 24, 24', 32, 32', 48) und jeweils zwei der stromabwärts zu positionierenden Wandler (12a, 12b, 12c, 12d, 24, 24', 32, 32', 48) auf dem Objekt (2) gegenüberliegend angeordnet sind und

- die Positionen der stromaufwärts zu positionierenden Wandler (10a, 10b, 10c, 10d, 24, 24', 32, 32', 48) gegenüber den Positionen der stromabwärts zu positionierenden Wandler (12a, 12b, 12c, 12d, 24, 24', 32, 32', 48) nur in Längsrichtung (L) des Objekts (2) variiert sind,

- weiterhin umfassend eine Steuereinheit, die, insbesondere anhand bestimmter Konfigurationsparameter, die für die jeweilige Messmethode notwendigen Wandler (10a, 10b, 10c, 10d, 12a, 12b, 12c, 12d, 24, 24', 32, 32', 48) steuert, sowie

- eine Auswerteeinheit, die die aus den empfangenen Signalen erzeugten Daten auswertet,

**dadurch gekennzeichnet, dass** die Steuereinheit sowie die Auswerteeinheit dazu eingerichtet sind, die Schritte des Verfahrens nach einem der Ansprüche 1 bis 23 auszuführen.

25. Vorrichtung nach Anspruch 24, **dadurch gekennzeichnet, dass** zumindest die entlang eines der Objektquerschnitte angeordneten EMAT-Wandler (10a, 10b, 10c, 10d, 12a, 12b, 12c, 12d, 24, 24', 32, 32', 48) in Ihrer Gesamtheit das Objekt (2) in Umfangsrichtung zumindest im Wesentlichen abdecken.

26. Vorrichtung nach Anspruch 24 oder 25, **dadurch gekennzeichnet, dass** die Vorrichtung entlang eines ersten Objektquerschnitts und/oder eines zweiten Objektquerschnitts jeweils zumindest sechs, vorzugsweise zumindest acht, insbesondere maximal 40, an oder nahe der Objektwand (4) zu positionierende EMAT-Wandler (44a, 44b, 44c, 44d, 44e, 44f, 44g, 44h, 48) umfasst.

27. Vorrichtung nach einem der Ansprüche 24 bis 26, **dadurch gekennzeichnet, dass** die Vorrichtung ferner zumindest einen das Objekt (2) im Wesentlichen vollumfänglich umfassenden EMAT-Wandler (42, 50) umfasst.

28. Vorrichtung nach einem der Ansprüche 24 bis 27, **dadurch gekennzeichnet, dass** die Vorrichtung zumindest zwei vollumfänglich ausgebildete EMAT-Wandler (42, 50) umfasst, wobei ein erster vollumfänglich ausgebildeter EMAT-Wandler (42, 50) stromaufwärts der zumindest vier stromaufwärts entlang eines ersten Objektquerschnitts an oder nahe der Objektwand (4) zu positionierende EMAT-Wandler (10a, 10b, 10c, 10d, 24, 24', 32, 32', 48) angeordnet ist und ein zweiter vollumfänglich ausgebildeter EMAT-Wandler (42, 50) stromabwärts der zumindest vier stromabwärts entlang eines zweiten Objektquerschnitts an oder nahe der Objektwand (4) zu positionierende EMAT-Wandler (12a, 12b, 12c, 12d, 24, 24', 32, 32', 48) angeordnet ist.

29. Vorrichtung nach einem der Ansprüche 24 bis 28, **dadurch gekennzeichnet, dass** zumindest einer der Wandler (10a, 10b, 10c, 10d, 12a, 12b, 12c, 12d, 24, 24', 32, 32', 42, 44a, 44b, 44c, 44d, 44e, 44f, 44g, 44h, 48, 50) als zumindest zwei zueinander räumlich versetze Spulen (53, 54) umfassender Phased-Array-Wandler ausgebildet ist.

30. Vorrichtung nach einem der Ansprüche 24 bis 29, **dadurch gekennzeichnet, dass** in radialer Richtung (R) des Objekts (2) über zumindest einem ersten Wandler (10a, 10b, 10c, 10d, 12a, 12b, 12c, 12d, 24, 24', 32, 32', 42, 44a, 44b, 44c, 44d, 44e, 44f, 44g, 44h, 48, 50), der zur Erzeugung von Ultraschallwellen (26) mit einer ersten Wellenlänge ($\lambda$) ausgebildet ist, zumindest ein weiterer Wandler (10a, 10b, 10c, 10d, 12a, 12b, 12c, 12d, 24, 24', 32, 32', 42, 44a, 44b, 44c, 44d, 44e, 44f, 44g, 44h, 48, 50) angeordnet ist, der zur Erzeugung von Ultraschallwellen (26) mit einer anderen Wellenlänge ($\lambda$) ausgebildet ist.

31. Vorrichtung nach einem der Ansprüche 24 bis 30, **dadurch gekennzeichnet, dass** die Vorrichtung zumindest einen flexiblen Träger (46) aufweist, in dem die stromaufwärts zu positionierenden Wandler (10a, 10b, 10c, 10d, 24, 24', 32, 32', 48) und/oder die stromabwärts zu positionierenden Wandler (12a, 12b, 12c, 12d, 24, 24', 32, 32', 48) und/oder die vollumfänglich ausgebildeten EMAT-Wandler (42, 50) angeordnet sind.

32. Vorrichtung nach Anspruch 31, **dadurch gekennzeichnet, dass** der flexible Träger (46) als flexible Leiterplatte ausgebildet ist und die Spulen (52 54) zumindest eines der Wandler (10a, 10b, 10c, 10d, 12a, 12b, 12c, 12d, 24, 24', 32, 32', 42, 44a, 44b, 44c, 44d, 44e, 44f, 44g, 44h, 48, 50) auf einer flexiblen Leiterplatte, insbesondere im Kupferbild, aufgedruckt sind.

33. Vorrichtung nach einem der Ansprüche 24 bis 32, **dadurch gekennzeichnet, dass** die Spulen (52, 54) zumindest eines der Wandler (10a, 10b, 10c, 10d, 12a, 12b, 12c, 12d, 24, 24', 32, 32', 42, 44a, 44b, 44c, 44d, 44e, 44f, 44g, 44h, 48, 50) in radialer Richtung (R) des Objekts (2) übereinander angeordnet sind.

**Claims**

1. Method for non-invasively determining properties of a multiphase flow which comprises a liquid fraction (6), in particular comprising water and/or a hydrocarbon-containing liquid, and a gaseous fraction and flows through an electrically conductive object (2), preferably a pipe or a pipeline, wherein the properties of the multiphase flow comprise in particular at least the velocity of the gaseous fraction, the velocity of the liquid fraction (6), the flow cross-section fraction of the gaseous fraction and/or the water content in the liquid fraction (6), wherein in the method

   - using a single set-up having a plurality of EMAT transducers (10a, 10b, 10c, 10d, 12a, 12b, 12c, 12d, 24, 24', 32, 32', 42, 44a, 44b, 44c, 44d, 44e, 44f, 44g, 44h, 48, 50), in particular according to device Claim 24,
   - at least two signals are spatially correlated with one another for determining the velocity of the gaseous fraction,
   - at least one signal arising on the basis of a wave (28) reflected from a reflection source (22) in the multiphase flow is evaluated for determining the velocity of the liquid fraction (6) and/or at least one signal arising on the basis of a wave (27) transmitted upstream and coupled at least into a part of the multiphase flow and at least one signal arising on the basis of a wave (27) transmitted downstream and coupled at least into a part of the multiphase flow are evaluated, for determining the velocity of the liquid fraction (6), and
   - at least one signal arising on the basis of a wave (27) transmitted downstream or upstream and coupled at least into a part of the multiphase flow is evaluated for determining the flow cross-section fraction of the gaseous fraction and/or at least one signal arising on the basis of a part of a wave (26) transmitted downstream or upstream, said part propagating exclusively in the object wall (4), is evaluated for determining the flow cross-section fraction of the gaseous fraction.

2. Method according to Claim 1, **characterized in that** at least the flow rate of the liquid fraction (6) and/or the flow rate of the gaseous fraction are/is ascertained from at least two properties of the multiphase flow.

3. Method according to either of the preceding claims, **characterized in that** for determining the water content in the liquid fraction (6), at least one signal arising on the basis of a part of a wave (26) transmitted downstream or upstream, said part propagating exclusively in the object wall (4), and/or at least one signal arising on the basis of a wave (27) transmitted upstream or downstream and coupled at least into a part of the multiphase flow are/is evaluated.

4. Method according to Claim 3, **characterized in that** for determining the water content in the liquid fraction (6), at least one signal arising on the basis of a wave (27) transmitted upstream or downstream and coupled at least into a part of the multiphase flow and a signal arising on the basis of a further wave (27) transmitted in the respective other direction (upstream or downstream) and coupled at least into a part of the multiphase flow are evaluated.

5. Method according to any of the preceding claims, **characterized in that**, in particular for determining the flow cross-section fraction of the gaseous fraction and/or for determining the water content in the liquid fraction (6), the at least one signal arising on the basis of a part of a wave (26) transmitted upstream or downstream at a first position, said part propagating exclusively in the object wall (4), is received at a second position, which is spaced apart from the first position in the longitudinal direction (L) of the object (2), wherein the composition of that part of the multiphase flow which adjoins the object wall (4) is ascertained on the basis of the amplitude (A) of the signal.

6. Method according to any of the preceding claims, **characterized in that** in the case of a horizontally extending object (2), at least one portion of the waves (27, 28) transmitted upstream and/or downstream and coupled at least into a part of the multiphase flow has in each case at least one propagation path extending between a 3 o'clock position and a 9 o'clock position.

7. Method according to any of the preceding claims, **characterized in that**, in particular for determining the velocity of the gaseous fraction, at least two signals at two positions spaced apart from one another in the longitudinal direction (L) of the object (2) are correlated with one another.

8. Method according to any of the preceding claims, **characterized in that**, in particular for determining the velocity of the liquid fraction (6) and/or the velocity of the gaseous fraction, a frequency shift of the signal arising on the basis of a wave (28) reflected from a reflection source in the multiphase flow is evaluated.

9. Method according to any of the preceding claims, **characterized in that**, in particular for determining the velocity of the liquid fraction (6), periodically waves (26) are generated and the signals arising on the basis of reflected waves (28) are evaluated, wherein, from a shift of the temporal position of at least one signal attributed to a specific reflection source (22), at least the velocity of the reflection source (22) is ascertained.

10. Method according to any of the preceding claims, **characterized in that**, in particular for determining the velocity of the liquid fraction (6), at least one signal arising on the basis of a wave (28) reflected from a reflection surface extending perpendicular or parallel to the main flow direction (S) of the multiphase flow or perpendicular to the main propagation direction (AR) of the wave (27) coupled into the multiphase flow is evaluated.

11. Method according to any of the preceding claims, **characterized in that**, in particular for determining the velocity of the liquid fraction (6), the propagation time difference between the at least one signal arising on the basis of the wave (27) transmitted upstream and coupled into at least one part of the multiphase flow and the at least one signal arising on the basis of the wave (27) transmitted downstream and coupled into at least one part of the multiphase flow is evaluated.

12. Method according to any of the preceding claims, **characterized in that**, in particular for determining the flow cross-section fraction of the gaseous fraction, at least one signal arising on the basis of a wave (28) reflected from a reflection source (22) in the multiphase flow, said wave arising at least partly from the wave (27) transmitted upstream or downstream at a first position and coupled into at least one part of the multiphase flow, is received at a second position, which is spaced apart from the first position in the longitudinal direction (L) of the object (2).

13. Method according to Claim 12, **characterized in that** the wave (26) transmitted downstream or upstream at a first position is transmitted from a deepest circumferential position (6 o'clock position) and the signal arising on the basis of the wave (28) reflected from a reflection source (22) in the multiphase flow is received at a deepest circumferential position (6 o'clock position).

14. Method according to any of the preceding claims, **characterized in that**, in particular for determining the flow cross-section fraction of the gaseous fraction, at least one signal arising on the basis of a wave (27) transmitted through at least one part of the multiphase flow is evaluated, wherein the transmitted wave (27) arises at least partly from the wave (26) transmitted into the multiphase flow downstream or upstream.

15. Method according to any of the preceding claims, **characterized in that**, in particular for determining the flow cross-section fraction of the gaseous fraction, at least one signal arising from a wave (28) transmitted twice through at least one part of the multiphase flow is evaluated, wherein the wave (28) transmitted twice arises at least partly from the wave (26) transmitted into the multiphase flow downstream or upstream.

16. Method according to any of the preceding claims, **characterized in that**, in particular for determining the flow cross-section fraction of the gaseous fraction, the at least one wave (26) transmitted upstream or downstream is transmitted into the object wall (4) fully circumferentially along a first object cross-section and the signals arising on the basis of that part of said wave (26) which propagates exclusively in the object wall (4), at a second object cross-section spaced apart from the first object cross-section in the longitudinal direction (L) of the object (2), are received at at least two different circumferential positions.

17. Method according to any of the preceding claims, **characterized in that** at least one of the measurement methods is assigned specific configuration parameters on the basis of which the transducers (10a, 10b, 10c, 10d, 12a, 12b, 12c, 12d, 24, 24', 32, 32', 42, 44a, 44b, 44c, 44d, 44e, 44f, 44g, 44h, 48, 50) necessary for the measurement method are controlled.

18. Method according to any of the preceding claims, **characterized in that** at least one of the transducers (10a, 10b, 10c, 10d, 12a, 12b, 12c, 12d, 24, 24', 32, 32', 42, 44a, 44b, 44c, 44d, 44e, 44f, 44g, 44h, 48, 50) is used both as transmitter and as receiver.

19. Method according to any of the preceding claims, **characterized in that** at least one of the transducers (10a, 10b, 10c, 10d, 12a, 12b, 12c, 12d, 24, 24', 32, 32', 42, 44a, 44b, 44c, 44d, 44e, 44f, 44g, 44h, 48, 50) transmits directionally upstream and/or downstream.

20. Method according to any of the preceding claims, **characterized in that** at least one of the transducers (10a, 10b, 10c, 10d, 12a, 12b, 12c, 12d, 24, 24', 32, 32', 42, 44a, 44b, 44c, 44d, 44e, 44f, 44g, 44h, 48, 50) generates Lamb waves in the object wall (4).

21. Method according to any of the preceding claims, **characterized in that** for at least one property of the multiphase flow the measurement method determining it is selected depending on the ratio of gaseous fraction to liquid fraction (6) of the multiphase flow, in particular depending on the flow rate of the liquid fraction (6) and/or the flow rate of the gaseous fraction.

22. Method according to any of the preceding claims, **characterized in that** for determining at least one of the properties for at least one measurement method a plurality of individual measurements are carried out and evaluated, wherein in particular preferably a mean value and/or a maximum are/is ascertained.

23. Method according to any of the preceding claims, **characterized in that** for at least one measurement method a pulse repetition frequency of at least 200 Hz, preferably at least 400 Hz, particularly preferably at least 800 Hz, and a maximum of 5 kHz, is used.

24. Device for non-invasively determining properties of a multiphase flow which comprises a liquid fraction (6), in particular comprising water and/or a hydrocarbon-containing liquid, and a gaseous fraction and flows through an electrically conductive object (2), preferably a pipe or a pipeline, comprising

   - at least four EMAT transducers (10a, 10b, 10c, 10d, 24, 24', 32, 32', 48) to be positioned upstream along a first object cross-section at or near the object wall (4) and
   - at least four EMAT transducers (12a, 12b, 12c, 12d, 24, 24', 32, 32', 48) to be positioned downstream along a second object cross-section at or near the object wall (4),
   - wherein respectively two of the transducers (10a, 10b, 10c, 10d, 24, 24', 32, 32', 48) to be positioned upstream and respectively two of the transducers (12a, 12b, 12c, 12d, 24, 24', 32, 32', 48) to be positioned downstream are arranged opposite one another on the object (2), and
   - the positions of the transducers (10a, 10b, 10c, 10d, 24, 24', 32, 32', 48) to be positioned upstream are varied relative to the positions of the transducers (12a, 12b, 12c, 12d, 24, 24', 32, 32', 48) to be positioned downstream only in the longitudinal direction (L) of the object (2),
   - furthermore comprising a control unit, which, in particular on the basis of specific configuration parameters, controls the transducers (10a, 10b, 10c, 10d, 12a, 12b, 12c, 12d, 24, 24', 32, 32', 48) necessary for the respective measurement method, and
   - an evaluation unit, which evaluates the data generated from the received signals,

   **characterized in that** the control unit and the evaluation nit are configured to carry out the steps of the method according to any of Claims 1 to 23.

25. Device according to Claim 24, **characterized in that** at least the EMAT transducers (10a, 10b, 10c, 10d, 12a, 12b, 12c, 12d, 24, 24', 32, 32', 48) arranged along one of the object cross-sections in their entirety at least substantially cover the object (2) in a circumferential direction.

26. Device according to Claim 24 or 25, **characterized in that** the device comprises along a first object cross-section and/or a second object cross-section in each case at least six, preferably at least eight, in particular a maximum of 40, EMAT transducers (44a, 44b, 44c, 44d, 44e, 44f, 44g, 44h, 48) to be positioned at or near the object wall (4).

27. Device according to any of Claims 24 to 26, **characterized in that** the device furthermore comprises at least one EMAT transducer (42, 50) encompassing the object (2) substantially fully circumferentially.

28. Device according to any of Claims 24 to 27, **characterized in that** the device comprises at least two EMAT transducers (42, 50) embodied fully circumferentially, wherein a first EMAT transducer (42, 50) embodied fully circumferentially is arranged upstream of the at least four EMAT transducers (10a, 10b, 10c, 10d, 24, 24', 32, 32', 48) to be positioned upstream along a first object cross-section at or near the object wall (4), and a second EMAT transducer (42, 50) embodied fully circumferentially is arranged downstream of the at least four EMAT transducers (12a, 12b, 12c, 12d, 24, 24', 32, 32', 48) to be positioned downstream along a second object cross-section at or near the object wall (4).

29. Device according to any of Claims 24 to 28, **characterized in that** at least one of the transducers (10a, 10b, 10c, 10d, 12a, 12b, 12c, 12d, 24, 24', 32, 32', 42, 44a, 44b, 44c, 44d, 44e, 44f, 44g, 44h, 48, 50) is embodied as a phased array transducer comprising at least two coils (53, 54) which are spatially offset with respect to one another.

30. Device according to any of Claims 24 to 29, **characterized in that** in a radial direction (R) of the object (2) above at least one first transducer (10a, 10b, 10c, 10d, 12a, 12b, 12c, 12d, 24, 24', 32, 32', 42, 44a, 44b, 44c, 44d, 44e, 44f, 44g, 44h, 48, 50) configured for generating ultrasonic waves (26) having a first wavelength (λ), there is arranged at least one further transducer (10a, 10b, 10c, 10d, 12a, 12b, 12c, 12d, 24, 24', 32, 32', 42, 44a, 44b, 44c, 44d, 44e, 44f, 44g, 44h, 48, 50) configured for generating ultrasonic waves (26) having a different wavelength (λ).

31. Device according to any of Claims 24 to 30, **characterized in that** the device has at least one flexible carrier (46), in which are arranged the transducers (10a, 10b, 10c, 10d, 24, 24', 32, 32', 48) to be positioned upstream and/or the transducers (12a, 12b, 12c, 12d, 24, 24', 32, 32', 48) to be positioned downstream and/or the EMAT transducers (42, 50) embodied fully circumferentially.

32. Device according to Claim 31, **characterized in that** the flexible carrier (46) is configured as a flexible printed circuit board and the coils (52, 54) of at least one of the transducers (10a, 10b, 10c, 10d, 12a, 12b, 12c, 12d, 24, 24', 32, 32', 42, 44a, 44b, 44c, 44d, 44e, 44f, 44g, 44h, 48, 50) are imprinted on a flexible printed circuit board, in particular in the copper pattern.

33. Device according to any of Claims 24 to 32, **characterized in that** the coils (52, 54) of at least one of the transducers (10a, 10b, 10c, 10d, 12a, 12b, 12c, 12d, 24, 24', 32, 32', 42, 44a, 44b, 44c, 44d, 44e, 44f, 44g, 44h, 48, 50) are arranged one above another in a radial direction (R) of the object (2).

**Revendications**

1. Procédé de détermination non invasive de propriétés d'un flux polyphasique qui comprend une fraction liquide (6), comprenant notamment de l'eau et/ou un liquide contenant des hydrocarbures, et une fraction gazeuse et qui s'écoule à travers un objet électriquement conducteur (2), de préférence un tube ou une canalisation, les propriétés du flux polyphasique comprenant notamment au moins la vitesse de la fraction gazeuse, la vitesse de la fraction liquide (6), la proportion de la fraction gazeuse au niveau d'une section transversale d'écoulement et/ou la teneur en eau dans la fraction liquide (6), dans le procédé

   - à l'aide d'une configuration unique comprenant une pluralité de transducteurs EMAT (10a, 10b, 10c, 10d, 12a, 12b, 12c, 12d, 24, 24', 32, 32', 42, 44a, 44b, 44c, 44d, 44e, 44f, 44g, 44h, 48, 50), notamment selon la revendication de dispositif 24,
   - au moins deux signaux étant spatialement corrélés entre eux pour déterminer la vitesse de la fraction gazeuse,
   - au moins un signal obtenu sur la base d'une onde (28) réfléchie par une source de réflexion (22) située dans le flux polyphasique, et/ou au moins un signal obtenu sur la base d'une onde (27) émise vers l'amont et injectée par couplage au moins dans une partie du flux polyphasique et au moins un signal obtenu sur la base d'une onde (27) émise vers l'aval et injectée par couplage dans au moins une partie du flux polyphasique, étant évalués pour déterminer la vitesse de la fraction liquide (6), et
   - au moins un signal obtenu sur la base d'une onde (27) émise vers l'aval ou vers l'amont et injectée par couplage dans au moins une partie du flux polyphasique, et/ou au moins un signal obtenu sur la base d'une partie, se propageant exclusivement dans la paroi d'objet (4), d'une onde (26) émise vers l'aval ou vers l'amont, étant évalués pour déterminer la proportion de la fraction gazeuse au niveau d'une section transversale d'écoulement.

2. Procédé selon la revendication 1, **caractérisé en ce qu'**au moins le débit de la fraction liquide (6) et/ou le débit de la fraction gazeuse sont déterminés à partir d'au moins deux propriétés du flux polyphasique.

3. Procédé selon l'une des revendications précédentes, **caractérisé en ce qu'**au moins un signal obtenu sur la base d'une partie, se propageant exclusivement dans la paroi d'objet (4), d'une onde (26) émise vers l'aval ou vers l'amont, et/ou au moins un signal obtenu sur la base d'une onde (27) émise vers l'amont ou vers l'aval et injectée par couplage dans au moins une partie du flux polyphasé, sont évalués pour déterminer la teneur en eau dans la fraction liquide (6).

4. Procédé selon la revendication 3, **caractérisé en ce qu'**au moins un signal obtenu sur la base d'une onde (27) émise vers l'amont ou vers l'aval et injectée par couplage dans au moins une partie du flux polyphasique et un signal obtenu sur la base d'une autre onde (27) émise dans l'autre direction respective (vers l'amont ou vers l'aval) et injectée par couplage dans au moins une partie du flux polyphasique sont évalués pour déterminer la teneur en eau dans la fraction liquide (6).

**5.** Procédé selon l'une des revendications précédentes, **caractérisé en ce que**, notamment pour déterminer la proportion de la fraction gazeuse au niveau d'une section transversale d'écoulement et/ou pour déterminer la teneur en eau de la fraction liquide (6), l'au moins un signal obtenu sur la base d'une partie, se propageant exclusivement dans la paroi d'objet (4), d'une onde (26) émise à une première position en amont ou en aval est reçu à une deuxième position distante de la première position dans la direction longitudinale (L) de l'objet (2), la composition de la partie, adjacente à la paroi d'objet (4), du flux polyphasique étant déterminée sur la base de l'amplitude (A) du signal.

**6.** Procédé selon l'une des revendications précédentes, **caractérisé en ce que**, dans le cas d'un objet (2) s'étendant horizontalement, au moins une partie des ondes (27, 28), émises vers l'amont et/ou vers l'aval et injectées par couplage dans au moins partie du flux polyphasique, ont chacune au moins un chemin de propagation qui s'étend entre une position à 3 heures et une position à 9 heures.

**7.** Procédé selon l'une des revendications précédentes, **caractérisé en ce que**, notamment pour déterminer la vitesse de la fraction gazeuse, au moins deux signaux situés à deux positions distantes l'une de l'autre dans la direction longitudinale (L) de l'objet (2) sont corrélés entre eux.

**8.** Procédé selon l'une des revendications précédentes, **caractérisé en ce qu'**un décalage en fréquence du signal, obtenu sur la base d'une onde (28) réfléchie par une source de réflexion située dans le flux polyphasique, est évalué notamment pour déterminer la vitesse de la fraction liquide (6) et/ou la vitesse de la fraction gazeuse.

**9.** Procédé selon l'une des revendications précédentes, **caractérisé en ce que** des ondes (26) sont générées périodiquement et les signaux obtenus sur la base des ondes réfléchies (28) sont évalués notamment pour déterminer la vitesse de la fraction liquide (6), au moins la vitesse d'une source de réflexion déterminée (22) étant déterminée à partir d'un décalage de la position temporelle d'au moins un signal remontant vers la source de réflexion (22).

**10.** Procédé selon l'une des revendications précédentes, **caractérisé en ce qu'**au moins un signal, obtenu sur la base d'une onde réfléchie (28) par une surface de réflexion qui s'étend perpendiculairement ou parallèlement à la direction d'écoulement principale (S) du flux polyphasique ou perpendiculairement à la direction de propagation principale (AR) de l'onde (27) injectée par couplage dans le flux polyphasique, est évalué notamment pour déterminer la vitesse de la fraction liquide (6).

**11.** Procédé selon l'une des revendications précédentes, **caractérisé en ce que** la différence de temps de propagation entre l'au moins un signal, obtenu sur la base d'une onde (27) émise vers l'amont et injectée par couplage dans au moins une partie du flux polyphasique, et l'au moins un signal, obtenu sur la base d'une onde (27) émise vers l'aval et injectée par couplage dans au moins une partie du flux polyphasique, est évaluée notamment pour déterminer la vitesse de la fraction liquide (6).

**12.** Procédé selon l'une des revendications précédentes, **caractérisé en ce qu'**au moins un signal, obtenu sur la base d'une onde (28) qui est réfléchie par une source de réflexion (22) située dans le flux polyphasique et qui est obtenue au moins partiellement à partir d'une onde (27) émise vers l'aval ou vers l'amont à première position et injectée par couplage dans au moins une partie du flux polyphasique, est reçu à une deuxième position distante de la première position dans la direction longitudinale (L) de l'objet (2) notamment pour déterminer la proportion de la fraction gazeuse au niveau d'une section transversale d'écoulement.

**13.** Procédé selon la revendication 12, **caractérisé en ce que** l'onde (26), émise vers l'aval ou vers l'amont à une première position, est émise depuis une position circonférentielle la plus basse (position à 6 heures) et **en ce que** le signal, obtenu sur la base de l'onde (28) réfléchie par la source de réflexion (22) située dans le flux polyphasique, est reçu à une position circonférentielle la plus basse (position à 6 heures).

**14.** Procédé selon l'une des revendications précédentes, **caractérisé en ce qu'**au moins un signal, obtenu sur la base d'une onde (27) transmise à travers au moins une partie du flux polyphasique, est évalué notamment pour déterminer la proportion de la fraction gazeuse au niveau d'une section transversale d'écoulement, l'onde transmise (27) étant obtenue au moins en partie à partir de l'onde (26) émise vers l'aval ou vers l'amont dans le flux polyphasique.

**15.** Procédé selon l'une des revendications précédentes, **caractérisé en ce qu'**au moins un signal, obtenu à partir d'une onde (28) transmise deux fois à travers au moins une partie du flux polyphasique est évalué notamment pour déterminer la proportion de la fraction gazeuse au niveau d'une section transversale d'écoulement, l'onde transmise deux fois (28) étant obtenue au moins en partie à partir de l'onde (26) émise vers l'aval ou vers l'amont dans le flux polyphasique.

16. Procédé selon l'une des revendications précédentes, **caractérisé en ce que** l'au moins une onde émise vers l'amont ou vers l'aval (26) est émise le long d'un première section transversale d'objet sur toute la circonférence jusque dans la paroi d'objet (4) et les signaux, obtenus sur la base de la partie de cette onde (26) qui se propage exclusivement dans la paroi d'objet (4) sont reçus en au moins deux positions circonférentielles différentes au niveau d'une deuxième section transversale d'objet distante de la première section transversale d'objet dans la direction longitudinale (L) de l'objet (2), notamment pour déterminer la proportion de la fraction gazeuse au niveau d'une section transversale d'écoulement.

17. Procédé selon l'une des revendications précédentes, **caractérisé en ce qu'**au moins une des méthodes de mesure est associée à des paramètres de configuration déterminés en fonction desquels les transducteurs (10a, 10b, 10c, 10d, 12a, 12b, 12c, 12d, 24, 24', 32, 32', 42, 44a, 44b, 44c, 44d, 44e, 44f, 44g, 44h, 48, 50) nécessaires à la méthode de mesure sont commandés.

18. Procédé selon l'une des revendications précédentes, **caractérisé en ce qu'**au moins un des transducteurs (10a, 10b, 10c, 10d, 12a, 12b, 12c, 12d, 24, 24', 32, 32', 42, 44a, 44b, 44c, 44d, 44e, 44f, 44g, 44h, 48, 50) est utilisé aussi bien comme émetteur que comme récepteur.

19. Procédé selon l'une des revendications précédentes, **caractérisé en ce qu'**au moins un des transducteurs (10a, 10b, 10c, 10d, 12a, 12b, 12c, 12d, 24, 24', 32, 32', 42, 44a, 44b, 44c, 44d, 44e, 44f, 44g, 44h, 48, 50) émet en étant dirigé vers l'amont et/ou vers l'aval.

20. Procédé selon l'une des revendications précédentes, **caractérisé en ce qu'**au moins un des transducteurs (10a, 10b, 10c, 10d, 12a, 12b, 12c, 12d, 24, 24', 32, 32', 42, 44a, 44b, 44c, 44d, 44e, 44f, 44g, 44h, 48, 50) génère des ondes Lamb dans la paroi d'objet (4).

21. Procédé selon l'une des revendications précédentes, **caractérisé en ce que**, en ce qui concerne au moins une propriété du flux polyphasique, la méthode de mesure déterminant celle-ci est sélectionnée en fonction du rapport fraction gazeuse à fraction liquide (6) du flux polyphasique, en notamment en fonction du débit de la fraction liquide (6) et/ou du débit de la fraction gazeuse.

22. Procédé selon l'une des revendications précédentes, **caractérisé en ce qu'**une pluralité de mesures individuelles sont réalisées et évaluées pour déterminer au moins une des propriétés pour au moins une méthode de mesure, notamment de préférence une valeur moyenne et/ou une valeur maximale étant déterminée.

23. Procédé selon l'une des revendications précédentes, **caractérisé en ce qu'**une fréquence de répétition d'impulsions d'au moins 200 Hz, de préférence d'au moins 400 Hz, de manière particulièrement préférée d'au moins 800 Hz, et au maximum de 5 kHz est utilisée pour au moins une méthode de mesure.

24. Dispositif de détermination non invasive de propriétés d'un flux polyphasique qui comprend une fraction liquide (6), comprenant notamment de l'eau et/ou un liquide contenant des hydrocarbures, et une fraction gazeuse et qui s'écoule à travers un objet électriquement conducteur (2), de préférence un tube ou une canalisation, ledit dispositif comprenant

- au moins quatre transducteurs EMAT (10a, 10b, 10c, 10d, 24, 24', 32, 32', 48) destinés à être positionnés en amont le long d'une première section transversale d'objet au niveau ou à proximité de la paroi d'objet (4) et
- au moins quatre transducteurs EMAT (12a, 12b, 12c, 12d, 24, 24', 32, 32', 48) destinés à être positionnés en aval le long d'une deuxième section transversale d'objet au niveau ou à proximité de la paroi d'objet (4),
- deux des transducteurs (10a, 10b, 10c, 10d, 24, 24', 32, 32', 48) destinés être positionnés en amont et deux des transducteurs (12a, 12b, 12c, 12d, 24, 24', 32, 32', 48) destinés à être positionnés en aval étant à chaque fois disposés face à face sur l'objet (2) et
- les positions des transducteurs (10a, 10b, 10c, 10d, 24, 24', 32, 32', 48) destinés à être positionnés en amont par rapport aux positions des transducteurs (12a, 12b, 12c, 12d, 24, 24', 32, 32', 48) destinés à être positionnés en aval ne variant que dans la direction longitudinale (L) de l'objet (2),
- ledit dispositif comprenant en outre une unité de commande qui commande les transducteurs (10a, 10b, 10c, 10d, 12a, 12b, 12c, 12d, 24, 24', 32, 32', 48) nécessaires à la méthode de mesure respective, notamment à l'aide de paramètres de configuration déterminés, et

- une unité d'évaluation qui évalue les données générées à partir des signaux reçus,

**caractérisé en ce que** l'unité de commande et l'unité d'évaluation sont conçues pour réaliser les étapes du procédé selon l'une des revendications 1 à 23.

25. Dispositif selon la revendication 24, **caractérisé en ce qu'**au moins les transducteurs EMAT (10a, 10b, 10c, 10d, 12a, 12b, 12c, 12d, 24, 24', 32, 32', 48) disposés le long d'une des sections transversales d'objet recouvrent au moins sensiblement intégralement l'objet (2) dans la direction circonférentielle.

26. Dispositif selon la revendication 24 ou 25, **caractérisé en ce que** le dispositif comprend au moins six, de préférence au moins huit, notamment au maximum 40, transducteurs EMAT (44a, 44b, 44c, 44d, 44e, 44f, 44g, 44h, 48), destinés à être positionnés au niveau ou à proximité de la paroi d'objet (4), le long d'une première section transversale d'objet et/ou une deuxième section transversale d'objet.

27. Dispositif selon l'une des revendications 24 à 26, **caractérisé en ce que** le dispositif comprend en outre au moins un transducteur EMAT (42, 50) qui englobe l'objet (2) sensiblement sur toute la circonférence.

28. Dispositif selon l'une des revendications 24 à 27, **caractérisé en ce que** le dispositif comprend au moins deux transducteurs EMAT (42, 50) de conception circonférentielle, un premier transducteur EMAT (42, 50) de conception circonférentielle étant disposé en amont des au moins quatre transducteurs EMAT (10a, 10b, 10c, 10d, 24, 24', 32, 32', 48) destinés à être positionnés en amont le long d'une première section transversale d'objet au niveau ou à proximité de la paroi d'objet (4) et un deuxième transducteur EMAT (42, 50) de conception circonférentielle étant disposé en aval des au moins quatre transducteurs EMAT (12a, 12b, 12c, 12d, 24, 24', 32, 32', 48) destinés à être positionnés en aval le long d'une deuxième section transversale d'objet au niveau ou à proximité de la paroi d'objet (4).

29. Dispositif selon l'une des revendications 24 à 28, **caractérisé en ce qu'**au moins un des transducteurs (10a, 10b, 10c, 10d, 12a, 12b, 12c, 12d, 24, 24', 32, 32', 42, 44a, 44b, 44c, 44d, 44e, 44f, 44g, 44h, 48, 50) est conçu comme un transducteur multiéléments comprenant au moins deux bobines (53, 54) décalées spatialement l'une de l'autre.

30. Dispositif selon l'une des revendications 24 à 29, **caractérisé en ce que** dans la direction radiale (R) de l'objet (2), au-dessus d'au moins un premier transducteur (10a, 10b, 10c, 10d, 12a, 12b, 12c, 12d, 24, 24', 32, 32', 42, 44a, 44b, 44c, 44d, 44e, 44f, 44g, 44h, 48, 50) qui est conçu pour générer des ondes ultrasonores (26) d'une première longueur d'onde ($\lambda$), est disposé au moins un autre transducteur (10a, 10b, 10c, 10d, 12a, 12b, 12c, 12d, 24, 24', 32, 32', 42, 44a, 44b, 44c, 44d, 44e, 44f, 44g, 44h, 48, 50) qui est conçu pour générer des ondes ultrasonores (26) d'une autre longueur d'onde ($\lambda$).

31. Dispositif selon l'une des revendications 24 à 30, **caractérisé en ce que** le dispositif comporte au moins un support souple (46) dans lequel sont disposés les transducteurs (10a, 10b, 10c, 10d, 24, 24', 32, 32', 48) destinés à être positionnés en amont et/ou les transducteurs (12a, 12b, 12c, 12d, 24, 24', 32, 32', 48) destinés à être positionnés en aval et/ou les transducteurs EMAT (42, 50) de conception circonférentielle.

32. Dispositif selon la revendication 31, **caractérisé en ce que** le support souple (46) est réalisé sous la forme d'un circuit imprimé souple et les bobines (52, 54) d'au moins un des transducteurs (10a, 10b, 10c, 10d, 12a, 12b, 12c, 12d, 24, 24', 32, 32', 42, 44a, 44b, 44c, 44d, 44e, 44f, 44g, 44h, 48, 50) sont imprimés sur un circuit imprimé souple, en particulier dans l'image de cuivre.

33. Dispositif selon l'une des revendications 24 à 32, **caractérisé en ce que** les bobines (52, 54) d'au moins un des transducteurs (10a, 10b, 10c, 10d, 12a, 12b, 12c, 12d, 24, 24', 32, 32', 42, 44a, 44b, 44c, 44d, 44e, 44f, 44g, 44h, 48, 50) sont disposées les unes au-dessus des autres dans la direction radiale (R) de l'objet (2).

Fig. 1

Fig. 2

Fig. 3

EP 3 867 636 B1

EP 3 867 636 B1

Fig. 4

EP 3 867 636 B1

Fig. 5

Fig. 6

EP 3 867 636 B1

Fig. 7

Fig. 8

Fig. 9

Fig. 10

Fig. 11

EP 3 867 636 B1

*Fig. 12*

Fig. 13

Fig. 14

46

48 48 48 48

Fig. 15

EP 3 867 636 B1

46

| 50 |
|----|

| 48 | 48 | 48 | 48 | 48 | 48 | 48 | 48 |

*Fig. 16*

EP 3 867 636 B1

48

W

52

54

$\lambda$

$\lambda/4$

W'

_Fig. 17_

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2018175503 A2
- EP 2913641 A2